(19) Europäisches Patentamt
European Patent Office
Office européen des brevets

(11) Veröffentlichungsnummer: **0 472 925 B1**

(12) **EUROPÄISCHE PATENTSCHRIFT**

(45) Veröffentlichungstag der Patentschrift: **31.08.94**

(21) Anmeldenummer: **91112637.3**

(22) Anmeldetag: **27.07.91**

Die Akte enthält technische Angaben, die nach dem Eingang der Anmeldung eingereicht wurden und die nicht in dieser Patentschrift enthalten sind.

(51) Int. Cl.[5]: **C07D 401/12**, A01N 43/54, //(C07D401/12,239:00,213:00), (C07D401/12,251:00,213:00)

# (54) Substituierte Pyridine.

(30) Priorität: **10.08.90 DE 4025338**

(43) Veröffentlichungstag der Anmeldung: **04.03.92 Patentblatt 92/10**

(45) Bekanntmachung des Hinweises auf die Patenterteilung: **31.08.94 Patentblatt 94/35**

(84) Benannte Vertragsstaaten: **BE CH DE ES FR GB IT LI NL**

(56) Entgegenhaltungen:
**EP-A- 0 242 081**
**EP-A- 0 372 329**
**EP-A- 0 374 839**
**EP-A- 0 402 751**
**EP-A- 0 435 186**

(73) Patentinhaber: **BAYER AG**
**D-51368 Leverkusen (DE)**

(72) Erfinder: **Drewes, Mark Wilhelm, Dr.**
**Grünstrasse 30**
**W-4018 Langenfeld (DE)**
Erfinder: **Müller, Peter, Dr.**
**Talstrasse 54**
**W-4018 Langenfeld (DE)**
Erfinder: **Santel, Hans-Joachim, Dr.**
**Grünstrasse 9a**
**W-5090 Leverkusen 1 (DE)**
Erfinder: **Lürssen, Klaus, Dr.**
**August-Kierspel-Strasse 145**
**W-5060 Bergisch Gladbach 2 (DE)**
Erfinder: **Schmidt, Robert R., Dr.**
**Im Waldwinkel 110**
**W-5060 Bergisch Gladbach 2 (DE)**

EP 0 472 925 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Pyridine, Verfahren sowie neue Zwischenprodukte zu ihrer Herstellung und ihre Verwendung als Herbizide.

Eine Reihe von substituierten Pyridinen mit herbiziden Eigenschaften ist bereits bekannt oder Gegenstand allgemeiner Patentansprüche (vgl. EP-A 249707, EP-A 360 163). Weiterhin werden in der EP-A 0 374 839 2-(4,6-Dimethoxy-2-pyrimidinyloxy)benzaldoxime und in der EP-A-0 435 186 Pyridyloxypyrimidine mit jeweils herbiziden Eigenschaften beschrieben.

Verbindungen aus den genannten Publikationen haben jedoch bisher keine größere Bedeutung erlangt.

Es wurden nun die neuen substituierten Pyridine der allgemeinen Formel (I) gefunden,

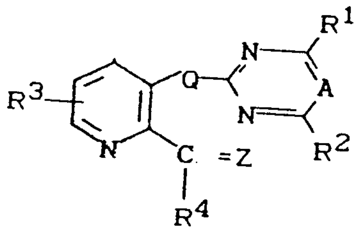

(I)

A für Stickstoff oder eine C-X-Gruppierung steht, wobei X für Wasserstoff oder Halogen steht,

Q für Sauerstoff oder Schwefel steht,

$R^1$ und $R^2$ gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen,

$R^3$ für Wasserstoff, Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy, steht,

$R^4$ für Wasserstoff oder $C_1$-$C_4$-Alkyl steht und

Z für eine der nachstehenden Gruppierungen steht:
N-$R^5$ oder

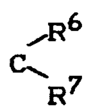

wobei

$R^5$ für Wasserstoff, Hydroxy, Amino oder für jeweils gegebenenfalls durch Halogen substituiertes $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkyl-carbonylamino, $C_1$-$C_6$-Alkoxy-carbonylamino, $C_1$-$C_6$-Alkyl-sulfonylamino, oder für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenoxy, Phenyl-$C_1$-$C_4$-alkoxy, Phenylamino, Phenyl-$C_1$-$C_4$-alkylamino, N-($C_1$-$C_4$-alkyl)-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Halogen, Cyano, Carboxy, $C_1$-$C_6$-Alkoxy-carbonyl, $C_1$-$C_6$-Alkyl-carbonylamino oder Di-($C_1$-$C_4$-alkoxy)-phosphoryl steht und

$R^7$ für Formyl, Cyano, Carboxy, Hydroxymethyl, Carbamoyl oder für jeweils gegebenenfalls durch Halogen, Carboxy oder $C_1$-$C_4$-Alkoxy-carbonyl substituiertes $C_1$-$C_6$-Alkoxy-carbonyl, $C_5$-$C_6$-Cycloalkyloxy-carbonyl, $C_1$-$C_6$-Alkylthio-carbonyl, $C_1$-$C_6$-Alkylamino-carbonyl oder $C_5$-$C_6$-Cycloalkylamino-carbonyl, für Di-($C_1$-$C_2$-alkyl)-amino-carbonyl, für $C_1$-$C_4$-Alkylamino-carbonyl-$C_1$-$C_4$-alkoxy-carbonyl, für Di-($C_1$-$C_2$-alkyl)-amino-carbonyl-$C_1$-$C_4$-alkoxy-carbonyl, für Phenylaminocarbonyl-$C_1$-$C_4$-alkoxy-carbonyl, für N-Methyl-N-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthio-carbonyl, Phenylaminocarbonyl, Phenyl-$C_1$-$C_4$-alkylamino-carbonyl, N-($C_1$-$C_4$-Alkyl)-N-phenylamino-carbonyl oder Phenylhydrazinocarbonyl oder $C_1$-$C_4$-Alkylhydrazinocarbonyl, für Phthalimidoxycarbonyl oder zusammen mit $R^6$ für die Gruppierung $-CO-O-(CH_2)_n-$steht, wobei

n für die Zahlen 1 bis 4 steht.

Man erhält die neuen substituierten Pyridine der allgemeinen Formel (I), wenn man substituierte Pyridine der allgemeinen Formel (Ia)

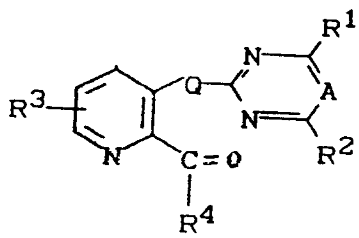

(Ia)

in welcher

A, Q, $R^1$, $R^2$, $R^3$ und $R^4$ die oben angegebene Bedeutung haben,

mit Amino- oder Methylen-Verbindungen der allgemeinen Formel (III)

$H_2Z$ (III)

in welcher

Z die oben angegebene Bedeutung hat,

gegebenenfalls in Gegenwart eines Reaktionshilfsmittels und gegebenenfalls in Gegenwart eines Verdünnungsmittels umsetzt und gegebenenfalls die hierbei erhaltenen Produkte anschließend nach üblichen Methoden in andere Derivate gemäß der Definition der Verbindungen der Formel (I) umwandelt.

Die neuen substituierten Pyridine der allgemeinen Formel (I) zeichnen sich durch starke Herbizidwirkung aus.

Bevorzugte Substituenten bzw. Bereiche der in den oben und nachstehend erwähnten Formeln aufgeführten Reste werden im folgenden erläutert:

Alkyl steht in den allgemeinen Formeln einzeln oder in zusammengesetzten Resten wie z.B. Alkylamino, Alkoxy-carbonylalkyl, für Alkyl mit vorzugsweise 1 bis 6, besonders bevorzugt 1 bis 4 und insbesondere 1 bis 2 Kohlenstoffatomen. Beispielhaft und vorzugsweise seien genannt Methyl, Ethyl, n- und iso-Propyl, n-, i-, s- und tert.-Butyl.

Alkoxy und Alkylthio stehen in den allgemeinen Formeln einzeln oder in zusammengesetzten Resten wie z.B. Alkoxycarbonyl, Alkylthiocarbonylalkyl für Alkoxy bzw. Alkylthio mit vorzugsweise 1 bis 6, besonders bevorzugt 1 bis 4 und insbesondere bevorzugt 1 bis 2 Kohlenstoffatomen. Beispielhaft und vorzugsweise seien Methoxy, Ethoxy, n- und i-Propoxy, n-, i-, s- und tert.-Butoxy, Methylthio, Ethylthio, n- und i-Propylthio, n-, i-, s- und tert.-Butylthio genannt.

Alkenyl und Alkinyl stehen in den allgemeinen Formeln einzeln oder in zusammengesetzten Resten für Alkenyl bzw. Alkinyl mit vorzugsweise 3 bis 6, besonders bevorzugt mit 3 oder 4 Kohlenstoffatomen. Beispielhaft und vorzugsweise seien genannt Allyl und Propargyl.

Halogenalkyl, Halogenalkoxy und Halogenalkylthio stehen in den allgemeinen Formeln für geradkettiges oder verzweigtes Halogenalkyl, Halogenalkoxy bzw. Halogenalkylthio mit 1 bis 4 und bevorzugt mit 1 oder 2 Kohlenstoffatomen und jeweils 1 bis 9 und bevorzugt 1 bis 5, gleichen oder verschiedenen Halogenatomen wie unter Halogen definiert; beispielhaft und vorzugsweise seien genannt: Fluormethyl, Chlormethyl, Brommethyl, Fluorethyl, Chlorethyl, Bromethyl, Fluor-n-propyl, Chlor-n-propyl, Dichlormethyl, Difluorethyl, Trifluorethyl, Trichlorethyl, Trifluorchlorethyl, Chlorbutyl, Fluorbutyl und insbesondere Difluormethyl, Trifluormethyl, Trichlormethyl, Dichlorfluor-methyl und Chlordifluor-methyl sowie die diesen entsprechenden Halogenalkoxyreste bzw. Halogenalkylthioreste.

Die hier aufgeführten Definitionen gelten in entsprechender Weise auch für die im folgenden aufgeführten bevorzugten Kombinationen von Resten.

Die in der Definition der erfindungsgemäßen Verbindungen aufgeführten aliphatischen Kohlenwasserstoffreste (z.B. Alkyl, Alkenyl, Alkinyl), auch in Kombination mit Heteroatomen (z.B. in Alkoxy, Alkylthio, Alkylamino) oder in Zusammensetzungen wie z.B. Halogenalkyl, Halogenalkoxy, sind jeweils geradkettig oder verzweigt.

Halogen steht im allgemeinen für Fluor, Chlor, Brom oder Jod, vorzugsweise für Fluor, Chlor oder Brom, insbesondere für Fluor oder Chlor.

Die Erfindung betrifft vorzugsweise Verbindungen der Formel (I), in welcher

A für Stickstoff oder eine CH-Gruppierung steht,

Q für Sauerstoff steht,

$R^1$ für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino steht,

$R^2$ für Wasserstoff, Chlor, Methyl, Ethyl, Trifluormethyl, Methoxy, Ethoxy, Difluormethoxy, Methylthio, Methylamino, Ethylamino oder Dimethylamino steht,

$R^3$ für Wasserstoff, Hydroxy, Fluor, Chlor, Brom, Methyl, Ethyl, Trifluormethyl, Methoxy, Difluormethoxy, Trifluormethoxy
steht,

$R^4$ für Wasserstoff oder Methyl steht und

Z für eine der nachstehenden Gruppierungen steht:

N-$R^5$ oder

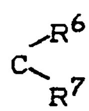 ,

wobei

$R^5$ für Wasserstoff, Hydroxy, Amino, für Methyl, Ethyl, n-Propyl, Isopropyl, n-Butyl, Isobutyl, sec-Butyl, tert-Butyl, Allyl, Propargyl, Methoxy, Ethoxy, n-Propoxy, Isopropoxy, n-Butoxy, Isobutoxy, sec-Butoxy, Allyloxy, Methoxycarbonylmethoxy, Ethoxycarbonylmethoxy, Methoxycarbonylethoxy, Ethoxycarbonylethoxy, Methylamino, Ethylamino, n-Propylamino, Isopropylamino, n-Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenyl, Benzyl, Phenoxy, Benzyloxy, Phenylamino, Benzylamino, N-Methyl-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Fluor, Chlor, Cyano, Carboxy, $C_1$-$C_4$-Alkoxy-carbonyl, $C_1$-$C_4$-Alkyl-carbonylamino, Dimethoxyphosphoryl oder Diethoxyphosphoryl steht und

$R^7$ für Formyl, Cyano, Carboxy, Hydroxymethyl, Carbamoyl, für jeweils gegebenenfalls durch Fluor, Chlor, Carboxy oder $C_1$-$C_4$-Alkoxy-carbonyl substituiertes $C_1$-$C_4$-Alkoxy-carbonyl, $C_5$-$C_6$-Cycloalkyloxy-carbonyl, $C_1$-$C_4$-Alkylthio-carbonyl, $C_1$-$C_4$-Alkylamino-carbonyl oder $C_5$-$C_6$-Cycloalkylamino-carbonyl, für Dimethylaminocarbonyl, für $C_1$-$C_4$-Alkylaminocarbonyl-$C_1$-$C_4$-alkoxy-carbonyl, für Dimethylaminocarbonyl-$C_1$-$C_4$-alkoxy-carbonyl, für N-Methyl-N-phenylaminocarbonyl-$C_1$-$C_4$-alkoxy-carbonyl, für jeweils gegebenenfalls durch Methyl und/oder Ethyl substituiertes Pyrrolidinyl-carbonyl, Piperidinyl-carbonyl, Morpholinyl-carbonyl oder Piperazinyl-carbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-N-phenylaminocarbonyl, Phenylhydrazinocarbonyl, für Phthalimidoxycarbonyl oder zusammen mit $R^6$ für die Gruppierung $-CO-O-CH_2CH_2-$ steht.

Ganz besonders bevorzugt werden die Verbindungen der Formel (I), worin

A für eine CH-Gruppierung steht,

Q für Sauerstoff steht,

$R^1$ für Methoxy steht,
$R^2$ für Methoxy steht,
$R^3$ für Wasserstoff oder Fluor steht,
$R^4$ für Wasserstoff steht und
Z die oben als insbesondere bevorzugt angegebene Bedeutung hat.

Die folgenden Bedeutungen für $R^5$ sind besonders hervorzuheben:
Wasserstoff$_3$ Hydroxy, Amino, Methyl, Ethyl, n-Propyl, i-Propyl, n-Butyl, i-Butyl, sec-Butyl, tert.-Butyl, Methylamino, Ethylamino, n-Propylamino, i-Propylamino, n-Butylamino, i-Butylamino, sec-Butylamino, tert.-Butylamino, Dimethylamino, Acetylamino, Propionylamino, Methoxycarbonylamino, Ethoxycarbonylamino, jeweils einfach bis dreifach gleich oder verschieden durch Fluor, Chlor, Nitro, Cyano, Methyl, Trifluormethyl, Methoxy, Trifluormethoxy substituiertes oder unsubstituiertes Phenyl, Benzyl, Phenylamino, Benzylamino, N-Methyl-N-phenylamino.

Die folgenden Bedeutungen für $R^6$ sind besonders hervorzuheben:
Wasserstoff, Cyano, Carboxy, $C_1$-$C_4$-Alkoxy-carbonyl.

Die folgenden Bedeutungen für $R^7$ sind besonders hervorzuheben:
Cyano, Carboxy, $C_1$-$C_4$-Alkoxy-carbonyl.

Verwendet man für das erfindungsgemäße Verfahren (a) zur Herstellung der Verbindungen der Formel (I) beispielsweise 3-(4,6-Dimethoxy-pyrimidin-2-yl-oxy)-2-hydroxymethyl-pyridin und Mangan(IV)-oxid als Ausgangsstoffe, so kann der Reaktionsablauf durch das folgende Formelschema wiedergegeben werden:

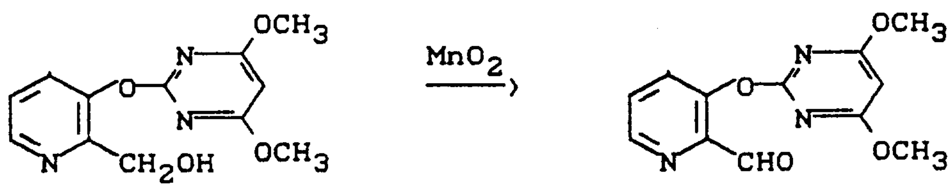


Verwendet man für das erfindungsgemäße Verfahren (b) zur Herstellung der Verbindungen der Formel (I) beispielsweise 3-(4-Methoxy-6-methyl-pyrimidin-2-yl-oxy)-2-formyl-pyridin und Hydroxylamin als Ausgangsstoffe, so kann der Reaktionsablauf durch das folgende Formelschema wiedergegeben werden:

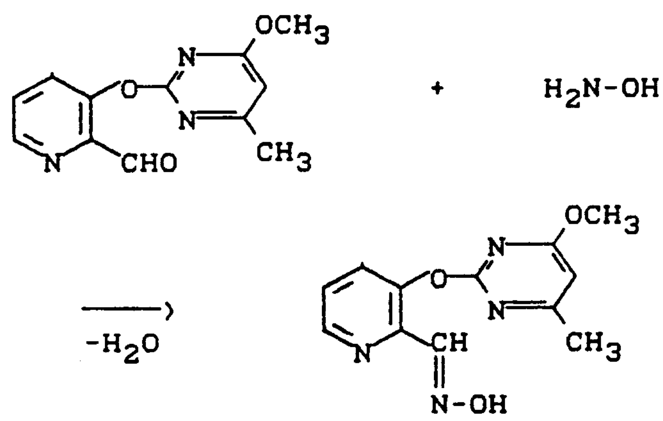


Die beim erfindungsgemäßen Verfahren (a) zur Herstellung von Verbindungen der Formel (I) als Ausgangsstoffe zu verwendenden Hydroxyalkylpyridine sind durch die Formel (II) allgemein definiert.

In Formel (II) haben A, Q, $R^1$, $R^2$, $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, Q, $R^1$, $R^2$, $R^3$ und $R^4$ angegeben wurden.

Als Beispiele für die Ausgangsstoffe der Formel (II) seien genannt:
3-(4,6-Dimethyl-pyrimidin-2-yl-oxy)-, 3-(4-Methoxy-6-methyl-pyrimidin-2-yl-oxy)-, 3-(4,6-Dimethoxy-pyrimidin-2-yl-oxy)-, 3-(4-Methoxy-6-trifluormethyl-pyrimidin-2-yl-oxy)-, 3-(4,6-Dimethyl-s-triazin-2-yl-oxy)-, 3-(4-Methoxy-6-methyl-s-triazin-2-yl-oxy)- und 3-(4,6-Dimethoxys-triazin-2-yl-oxy)-2-hydroxymethyl-pyridin.

Die Hydroxyalkylpyridine der Formel (II) sind noch nicht aus der Literatur bekannt und ebenfalls Gegenstand der vorliegenden Anmeldung. Man erhält die neuen Verbindungen der Formel (II), wenn man Pyridincarbonsäurederivate der allgemeinen Formel (IV)

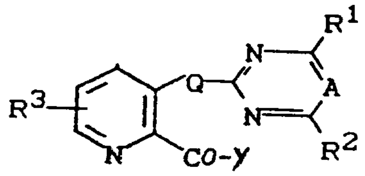

(IV)

in welcher
A, Q, $R^1$, $R^2$ und $R^3$ die oben angegebene Bedeutung haben und
Y für Halogen, Hydroxy oder Alkoxy steht,
mit Metallverbindungen der allgemeinen Formel (V)

$MR^4$ (V)

in welcher
$R^4$ die oben angegebene Bedeutung hat und
M für eine bei (gegebenenfalls komplexen) Metallhydriden oder metallorganischen Verbindungen übliche metallische oder metallhaltige Komponente steht,
gegebenenfalls in Gegenwart eines Verdünnungsmittels, wie z.B. Diethylether, Dimethoxyethan, Tetrahydrofuran, Methanol, Ethanol oder Isopropanol, bei Temperaturen zwischen -70 °C und +50 °C umsetzt und auf übliche Weise aufarbeitet (vgl. Herstellungsbeispiele).

In Formel (IV) haben A, Q, $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, Q, $R^1$, $R^2$ und $R^3$ angegeben wurden und Y steht vorzugsweise für Chlor, Hydroxy, Methoxy oder Ethoxy.

Als Beispiele für die Ausgangsstoffe der Formel (IV) seien genannt:
3-(4,6-Dimethyl-pyrimidin-2-yl-oxy)-, 3-(4-Methoxy-6-methyl-pyrimidin-2-yl-oxy)-, 3-(4,6-Dimethoxy-pyrimidin-2-yl-oxy)-, 3-(4-Methoxy-6-trifluormethyl-pyrimidin-2-yl-oxy)-, 3-(4,6-Dimethyl-s-triazin-2-yl-oxy)-, 3-(4-Methoxy-6-methyl-s-triazin-2-yl-oxy)- und 3-(4,6-Dimethoxy-s-triazin-2-yl-oxy)-pyridin-2-carbonsäure sowie die entsprechenden Carbonsäurechloride, die Methylester und die Ethylester.

Die Pyridincarbonsäurederivate der Formel (IV) sind bekannt und/oder können nach an sich bekannten Verfahren hergestellt werden (vgl. EP-A 249707).

In Formel (V) hat $R^4$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $R^4$ angegeben ist und
M steht vorzugsweise für ein Metall aus der Reihe Lithium, Natrium, Kalium, Magnesium, Calcium, Bor und Aluminium, an welches gegebenenfalls (soweit mehrwertig) zusätzlich Wasserstoff-, Alkalimetall- oder Halogenatome gebunden sind.

Als Beispiele für die Verbindungen der Formel (V) seien genannt:
Lithium-, Natrium-, Kalium-, Magnesium- und Calcium-hydrid, Lithium-, Natrium- und Kalium-tetrahydridoborat (-"borhydrid", -"boranat"), Lithium-, Natrium- und Kalium-tetrahydridoaluminat(-"alanat"), Methyllithium, Butyllithium, Methylmagnesium-chlorid, -bromid und -iodid, Ethyl-, Propyl- und Isopropylmagnesium-chlorid, -bromid und -iodid.

Die Verbindungen der Formel (V) sind bekannte Synthesechemikalien.

Das erfindungsgemäße Verfahren (a) wird unter Verwendung von Oxidationsmitteln durchgeführt. Als Oxidationsmittel werden hierbei vorzugsweise die üblicherweise für die Dehydrierung von Alkoholen zu Aldehyden oder Ketonen verwendeten Stoffe, wie z.B. Mangan(IV)-oxid ("Braunstein"), Dimethylsulfoxid/Oxalylchlorid (Swern-Reagenz), Chrom(VI)-oxid oder Natriumdichromat/Schwefelsäure, eingesetzt.

Das erfindungsgemäße Verfahren (a) zur Herstellung der neuen substituierten Pyridine der Formel (I) wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform,

Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Die Reaktionstemperaturen können bei dem erfindungsgemäßen Verfahren (a) in einem größeren Bereich variiert werden. Im allgemeinen arbeitet man bei Temperaturen zwischen -80 °C und +150 °C, vorzugsweise bei Temperaturen zwischen -60 °C und +100 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man auf 1 Mol Hydroxyalkylpyridin der Formel (II) im allgemeinen zwischen 1 und 50, vorzugsweise zwischen 1 und 25 Moläquivalenten, des Oxidationsmittels ein.

Im allgemeinen wird die Ausgangsverbindung der Formel (II) in einem geeigneten Verdünnungsmittel vorgelegt und das Oxidationsmittel langsam dazu gegeben. Das Reaktionsgemisch wird bei der erforderlichen Temperatur bis zum Ende der Umsetzung gerührt und auf übliche Weise aufgearbeitet (vgl. Herstellungsbeispiele).

Die beim erfindungsgemäßen Verfahren (b) zur Herstellung von Verbindungen der Formel (I) als Ausgangsstoffe zu verwendenden substituierten Pyridine sind durch die Formel (Ia) allgemein definiert.

In Formel (Ia) haben A, Q, $R^1$, $R^2$, $R^3$ und $R^4$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, Q, $R^1$, $R^2$, $R^3$ und $R^4$ angegeben wurden.

Die substituierten Pyridine der Formel (Ia) sind erfindungsgemäße, neue Verbindungen; sie können nach dem erfindungsgemäßen Verfahren (a) hergestellt werden.

Die beim erfindungsgemäßen Verfahren (b) zur Herstellung von Verbindungen der Formel (I) weiter als Ausgangsstoffe zu verwendenden Amino- oder Methylen-Verbindungen sind durch die Formel (III) allgemein definiert.

In Formel (III) hat Z mit Ausnahme von Sauerstoff vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Z angegeben wurde.

Als Beispiele für die Ausgangsstoffe der Formel (III) seien genannt:
Ammoniak, Hydroxylamin, Hydrazin, Methylamin, Ethylamin, Propylamin, Isopropylamin, Butylamin, Isobutylamin, sec-Butylamin, tert-Butylamin, Allylamin, Propargylamin, O-Methyl-, O-Ethyl-, O-Propyl-, O-Isopropyl-, O-Butyl-, O-Isobutyl- und O-sec-Butyl-hydroxylamin, O-Allyl-hydroxylamin, Aminooxyessigsäure-methylester und -ethylester, $\alpha$-Aminooxy-propionsäure-methylester und -ethylester, Methylhydrazin, Ethylhydrazin, Propylhydrazin, Isopropylhydrazin, Butylhydrazin, Isobutylhydrazin, sec-Butyl-hydrazin, tert-Butylhydrazin, N,N-Dimethylhydrazin, Acethydrazid, Propionylhydrazid, Methoxycarbonylhydrazin, Ethoxycarbonylhydrazin, Methylsulfonylhydrazin, Ethylsulfonylhydrazin, Phenylhydrazin, Benzoylhydrazin, Benzolsulfonsäurehydrazid, p-Toluolsulfonsäurehydrazid, Malonsäure, Cyanessigsäure, Malonsäuredinitril, Cyanessigsäure-methylester und -ethylester, Malonsäure-dimethylester und -diethylester, $\gamma$-Butyrolacton.

Die Ausgangsstoffe der Formel (III) sind bekannte Synthesechemikalien.

Das erfindungsgemäße Verfahren (b) zur Herstellung der neuen substituierten Pyridine der Formel (I) wird vorzugsweise unter Verwendung von Verdünnungsmitteln durchgeführt. Als Verdünnungsmittel kommen dabei neben Wasser praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Das erfindungsgemäße Verfahren (b) wird gegebenenfalls in Gegenwart eines Reaktionshilfsmittels durchgeführt. Geeignete Reaktionshilfsmittel sind Stoffe, die üblicherweise zur Steuerung und/oder Beschleunigung von Kondensationsreaktionen zwischen Carbonylverbindungen und Amino- oder Methylen-Verbindungen verwendet werden. Hierzu gehören insbesondere Stickstoffverbindungen, wie z.B. Ammoniumacetat, $\beta$-Alanin, Pyridin und Piperidin.

Die Reaktionstemperaturen können bei dem erfindungsgemäßen Verfahren (b) in einem größeren Bereich variiert werden. Im allgemeinen arbeitet man bei Temperaturen zwischen 0 °C und 150 °C,

vorzugsweise bei Temperaturen zwischen 10 °C und 120 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel, gegebenenfalls in Gegenwart eines Reaktionshilfmittels durchgeführt, und das Reaktionsgemisch wird mehrere Stunden bei der jeweils erforderlichen Temperatur gerührt. Die Aufarbeitung erfolgt bei dem erfindungsgemäßen Verfahren jeweils nach üblichen Methoden (vergleiche die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern sowohl im Vorauflauf- als auch im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden, Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl,

Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen infrage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor4-methylphenyl)-harnstoff (CHLORTOLURON); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); Methyl-2-[4,5-dihydro-4-methyl-4-(1-methylethyl)-5-oxo1H-imidazol-2-yl]-4(5)-methylbenzoat (IMAZAMETHABENZ); 2-[5-Methyl-5-(1-methylethyl)-4-oxo-2-imidazolin-2-yl]-3-chinolincarbonsäure (IMAZAQUIN); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)pyrazol-1-yl-methyl]-acetamid (METAZACHLOR); 2-Ethyl-6-methyl-N-(1-methyl-2-methoxyethyl)-chloracetanilid (METOLACHLOR); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor,

Herstellungsbeispiele:

Beispiel 1

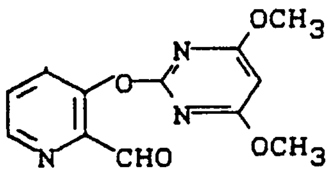

(Verfahren (a))

Eine Lösung von 8,0 g (0,03 Mol) 2-Hydroxymethyl-3-(4,6-dimethoxy-pyrimidin-2-yl-oxy)-pyridin in Chloroform (250 ml) wird zum Sieden erhitzt. Innerhalb von 4 Stunden werden 4 Portionen von je 15 g Mangan(IV)-oxid (insgesamt 0,69 Mol) dazu gegeben; das Gemisch wird insgesamt 5 Stunden unter Rückfluß gerührt. Anschließend wird filtriert und vom Filtrat das Lösungsmittel im Wasserstrahlvakuum sorgfältig abdestilliert.

Man erhält 7,3 g (92% der Theorie) 2-Formyl-3-(4,6-dimethoxy-pyrimidin-2-yl-oxy)-pyridin als festen Rückstand vom Schmelzpunkt 76 °C.

Beispiel 2:

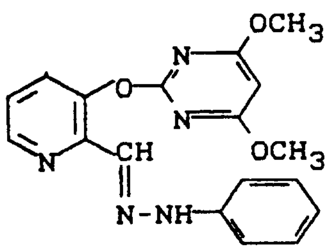

(Verfahren (b))

Eine Mischung aus 1,56 g (6 mMol) 3-(4,6-Dimethoxy-pyrimidin-2-yl-oxy)-pyridin-2-aldehyd, 0,66 g (6 mMol) Phenylhydrazin und 40 ml Toluol wird 1/2 Stunde bei 20 °C gerührt und anschließend eingeengt. Der Rückstand wird mit Petrolether zur Kristallisation gebracht und das kristalline Produkt durch Absaugen isoliert.

Man erhält 2,0 g (95% der Theorie) 3-(4,6-Dimethoxy-pyrimidin-2-yl-oxy)-pyridin-2-aldehyd-phenylhydrazon vom Schmelzpunkt 118 °C (Zers.).

Beispiel 3:

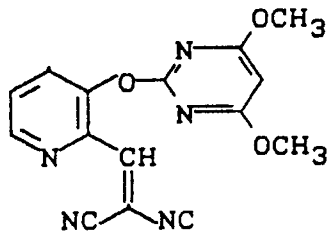

(Verfahren (b))

Eine Mischung aus 1,56 g (6 mMol) 3-(4,6-Dimethoxy-pyrimidin-2-yl-oxy)-pyridin-2-aldehyd, 0,40 g (6 mMol) Malonsäuredinitril, 0,2 g Ammoniumacetat und 80 ml Toluol wird 2 Stunden bei 20 °C gerührt. Anschließend wird eingeengt und der Rückstand mit Petrolether zur Kristallisation gebracht. Das kristalline Produkt wird durch Absaugen isoliert.

Man erhält 1,2 g (65% der Theorie) α-(3-(4,6-Dimethoxypyrimidin-2-yl-oxy)-pyridin-2-yl)-methylen-malonsäuredinitril vom Schmelzpunkt 120 °C (Zers.).

Analog zu den Beispielen 1 bis 3 sowie entsprechend der allgemeinen Beschreibung der erfindungsgemäßen Herstellungsverfahren können beispielsweise auch die in der nachstehenden Tabelle 1 aufgeführten Verbindungen der Formel (I) - bzw. der Formeln (IA) oder (IB) - hergestellt werden.

Tabelle 1: Beispiele für die Verbindungen der Formel (IA)

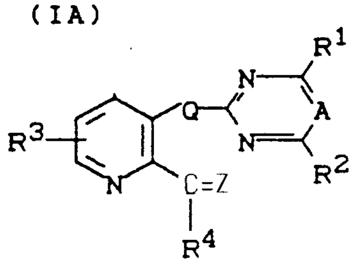

(IA)

| Bsp.-Nr. | A | Q | $R^1$ | $R^2$ | $R^3$ | $R^4$ | Z | Schmelzpunkt (°C) |
|---|---|---|---|---|---|---|---|---|
| 4 | CH | O | $OCH_3$ | $OCH_3$ | H | H | N-NH 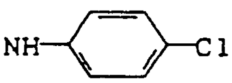 | 137 |
| 5 | CH | O | $OCH_3$ | $OCH_3$ | H | H | N-NH 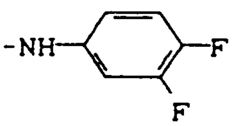 | 172 |
| 6 | CH | O | $OCH_3$ | $OCH_3$ | H | H | N-N 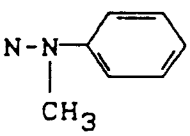 | amorph |
| 7 | CH | O | $OCH_3$ | $OCH_3$ | H | H | $N-NH-SO_2-CH_3$ | 112 |
| 8 | CH | O | $OCH_3$ | $OCH_3$ | H | H | N-NH 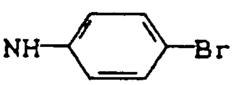 | 109 |
| 9 | CH | O | $OCH_3$ | $OCH_3$ | H | H | N-NH 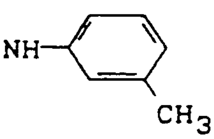 | 116 |
| 10 | CH | O | $OCH_3$ | $OCH_3$ | H | H | N-OH | 121 |

Tabelle 1 - Fortsetzung -

| Bsp.-Nr. | A | Q | $R^1$ | $R^2$ | $R^3$ | $R^4$ | Z | Schmelzpunkt (°C) |
|---|---|---|---|---|---|---|---|---|
| 11 | CH | O | $OCH_3$ | $OCH_3$ | H | H | 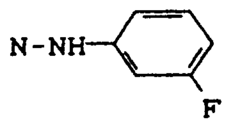 | 166 |
| 12 | CH | O | $OCH_3$ | $OCH_3$ | H | H | 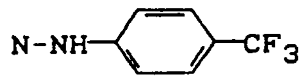 | 55 |
| 13 | CH | O | $OCH_3$ | $OCH_3$ | H | H | 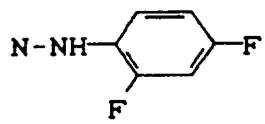 | 142 |
| 14 | CH | O | $OCH_3$ | $OCH_3$ | H | H | 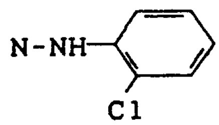 | 138 |
| 15 | CH | O | $OCH_3$ | $OCH_3$ | H | H | 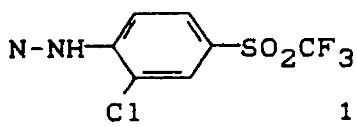 | 168 |
| 16 | CH | O | $OCH_3$ | $OCH_3$ | H | H | 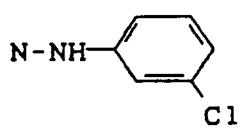 | 147 |
| 17 | CH | O | $OCH_3$ | $OCH_3$ | H | H | 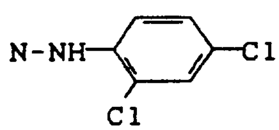 | 132 |
| 18 | CH | O | $OCH_3$ | $OCH_3$ | H | H | 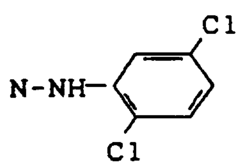 | 163 |

Tabelle 1 - Fortsetzung -

| Bsp.-Nr. | A | Q | $R^1$ | $R^2$ | $R^3$ | $R^4$ | Z | Schmelzpunkt (°C) |
|---|---|---|---|---|---|---|---|---|
| 19 | CH | O | $OCH_3$ | $OCH_3$ | H | H | 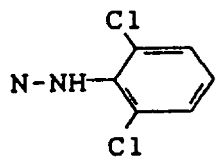 | 111 |
| 20 | CH | O | $OCH_3$ | $OCH_3$ | H | H | 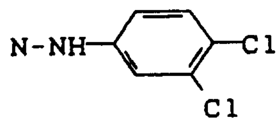 | 75 |
| 21 | CH | O | $OCH_3$ | $OCH_3$ | H | H | 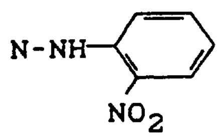 | 102 |
| 22 | CH | O | $OCH_3$ | $OCH_3$ | H | H | 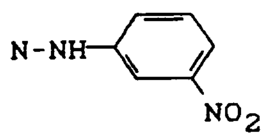 | 153 |
| 23 | CH | O | $OCH_3$ | $OCH_3$ | H | H | 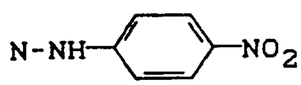 | 187 |
| 24 | CH | O | $OCH_3$ | $OCH_3$ | H | H | 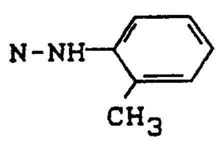 | 117 |
| 25 | CH | O | $OCH_3$ | $OCH_3$ | H | H | 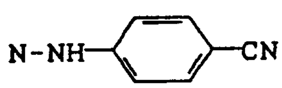 | 185 |
| 26 | CH | O | $OCH_3$ | $OCH_3$ | H | H | 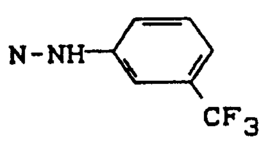 | 126 |

Tabelle 1 - Fortsetzung -

| Bsp.-Nr. | A | Q | $R^1$ | $R^2$ | $R^3$ | $R^4$ | Z | Schmelzpunkt (°C) |
|---|---|---|---|---|---|---|---|---|
| 27 | CH | O | $OCH_3$ | $OCH_3$ | H | H | $N-N(CH_3)_2$ | (amorph) |
| 28 | CH | O | $OCH_3$ | $OCH_3$ | H | H | $N-NH-CO-CH_3$ | 145 |
| 29 | CH | O | $OCH_3$ | $OCH_3$ | H | H | 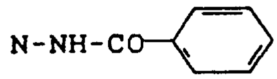 | 128 |
| 30 | CH | O | $OCH_3$ | $OCH_3$ | H | H | 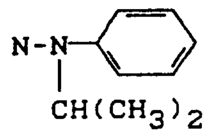 | 88 |
| 31 | CH | O | $OCH_3$ | $OCH_3$ | H | H | 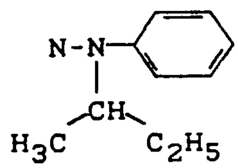 | 90 |
| 32 | CH | O | $OCH_3$ | $OCH_3$ | H | H | 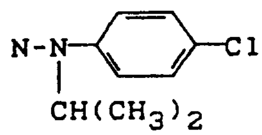 | 62 |
| 33 | CH | O | $OCH_3$ | $OCH_3$ | H | H | 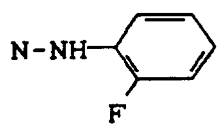 | 132 |

Ausgangsstoffe der Formel (II)

Beispiel (II-1)

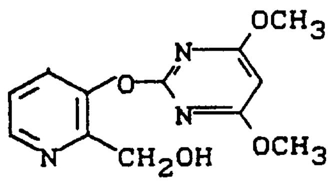

Eine Mischung aus 18,3 g (0,06 Mol) 3-(4,6-Dimethoxypyrimidin-2-yl-oxy)-pyridin-2-carbonsäure-ethylester und 200 ml Ethanol wird auf 0°C bis 5°C abgekühlt und unter Rühren mit 6,5 g (0,17 Mol) Natriumborhydrid portionsweise versetzt. Dann wird eine Lösung von 10 g Calciumchlorid in 60 ml Ethanol zugetropft und das Gemisch eine Stunde bei 0°C bis 5°C gerührt. Anschließend wird zum Entfernen des Borhydrid-Überschusses mit Salzsäure angesäuert und dann durch Zugabe von Natriumcarbonat wieder schwach alkalisch gestellt. Man extrahiert mit Diethylether, engt die Extraktionslösung ein, bringt den Rückstand durch Verreiben mit Diethylether/Petrolether zur Kristallisation und isoliert das Produkt durch Absaugen.

Man erhält 9,3 g (59% der Theorie) 2-Hydroxymethyl-3-(4,6-dimethoxy-pyrimidin-2-yl-oxy)-pyridin vom Schmelzpunkt 115°C.

Anwendungsbeispiele:

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant, Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

O % = keine Wirkung (wie unbehandelte Kontrolle)
100 % = totale Vernichtung

In diesem Test zeigen beispielsweise die Verbindungen gemäß den Herstellungsbeispielen (2) und (3) bei guter Verträglichkeit gegenüber Kulturpflanzen, wie z.B. Soja, starke Wirkung gegen Unkräuter.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im

Vergleich zur Entwicklung der unbehandelten Kontrolle. Es bedeuten:

O % = keine Wirkung (wie unbehandelte Kontrolle)

100 % = totale Vernichtung

In diesem Test zeigen beispielsweise die Verbindungen gemäß den Herstellungsbeispielen (1), (2) und (3) bei guter Verträglichkeit gegenüber Kulturpflanzen, wie z.B. Mais, starke Wirkung gegen Unkräuter.

**Patentansprüche**

**Patentansprüche für folgende Vertragsstaaten: BE, CH, DE, FR, GB, IT, LI, NL**

**1.** Substituierte Pyridine der allgemeinen Formel (I)

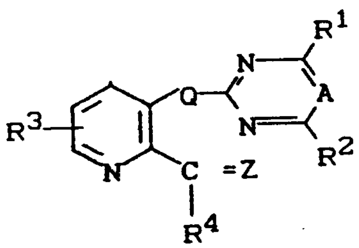

(I)

in welcher

| | |
|---|---|
| A | für Stickstoff oder eine C-X-Gruppierung steht, wobei X für Wasserstoff oder Halogen steht, |
| Q | für Sauerstoff oder Schwefel steht, |
| $R^1$ und $R^2$ | gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalyoxy, $C_1$-$C_4$-Alylthio, $C_1$-$C_4$-Alkylamino oder Di-($C_1$-$C_2$-alkyl)-amino stehen, |
| $R^3$ | für Wasserstoff, Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy steht, |
| $R^4$ | für Wasserstoff oder $C_1$-$C_4$-Alkyl steht und |
| Z | für eine der nachstehenden Gruppierungen steht: N-$R^5$ oder |

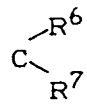

,

wobei

| | |
|---|---|
| $R^5$ | für Wasserstoff, Hydroxy, Amino oder für jeweils gegebenenfalls durch Halogen substituiertes $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkyl-carbonylamino, $C_1$-$C_6$-Alkoxy-carbonylamino, $C_1$-$C_6$-Alkyl-sulfonylamino, oder für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenoxy, Phenyl-$C_1$-$C_4$-alkoxy, Phenylamino, Phenyl-$C_1$-$C_4$-alkylamino, N-($C_1$-$C_4$-alkyl)-N-phenylamino, Pyridylamino, Pyrimidylamino, Pyridylcarbonylamino, Phenylcarbonylamino, Furylcarbonylamino, Thienylcarbonylamino oder Phenylsulfonylamino steht, |
| $R^6$ | für Wasserstoff, Halogen, Cyano, Carboxy, $C_1$-$C_6$-Alkoxy-carbonyl, $C_1$-$C_6$-Alkyl-carbonylamino oder Di-($C_1$-$C_4$-alkoxy)-phosphoryl steht und |
| $R^7$ | für Formyl, Cyano, Carboxy, Hydroxymethyl, Carbamoyl oder für jeweils gegebenenfalls durch Halogen, Carboxy oder $C_1$-$C_4$-Alkoxy-carbonyl substituiertes $C_1$-$C_6$-Alkoxy-carbonyl, $C_5$-$C_6$-Cycloalkyloxy-carbonyl, $C_1$-$C_6$-Alkylthio-carbonyl, $C_1$-$C_6$-Alkylamino-carbonyl oder $C_5$-$C_6$-Cycloalkylamino-carbonyl, für Di-($C_1$-$C_2$-alkyl)-amino-carbonyl, für $C_1$-$C_4$-Alkylamino-carbonyl-$C_1$-$C_4$-alkoxy-carbonyl, für Di-($C_1$-$C_2$-alkyl)-amino-car- |

bonyl-$C_1$-$C_4$-alkoxy-carbonyl, für Phenylaminocarbonyl-$C_1$-$C_4$-alkoxy-carbonyl, für N-Methyl-N-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthio-carbonyl, Phenylaminocarbonyl, Phenyl-$C_1$-$C_4$-alkylamino-carbonyl, N-($C_1$-$C_4$-Alkyl)-N-phenylamino-carbonyl oder Phenylhydrazinocarbonyl oder $C_1$-$C_4$-Alkylhydrazinocarbonyl, für Phthalimidoxycarbonyl oder zusammen mit $R^6$ für die Gruppierung $-CO-O-(CH_2)_n$-steht, wobei

n für die Zahlen 1 bis 4 steht.

**2.** Substituierte Pyridine der Formel (I) gemäß Anspruch 1, in welcher

A für Stickstoff oder eine CH-Gruppierung steht,

Q für Sauerstoff steht,

$R^1$ für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino steht,

$R^2$ für Wasserstoff, Chlor, Methyl, Ethyl, Trifluormethyl, Methoxy, Ethoxy, Difluormethoxy, Methylthio, Methylamino, Ethylamino oder Dimethylamino steht,

$R^3$ für Wasserstoff, Hydroxy, Fluor, Chlor, Brom, Methyl, Ethyl, Trifluormethyl, Methoxy, Difluormethoxy, Trifluormethoxy, steht,

$R^4$ für Wasserstoff oder Methyl steht und

Z für eine der nachstehenden Gruppierungen steht:

N-$R^5$ oder

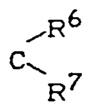 ,

wobei

$R^5$ für Wasserstoff, Hydroxy, Amino, für Methyl, Ethyl, n-Propyl, Isopropyl, n-Butyl, Isobutyl, sec-Butyl, tert-Butyl, Allyl, Propargyl, Methoxy, Ethoxy, n-Propoxy, Isopropoxy, n-Butoxy, Isobutoxy, sec-Butoxy, Allyloxy, Methoxycarbonylmethoxy, Ethoxycarbonylmethoxy, Methoxycarbonylethoxy, Ethoxycarbonylethoxy, Methylamino, Ethylamino, n-Propylamino, Isopropylamino, n-Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenyl, Benzyl, Phenoxy, Benzyloxy, Phenylamino, Benzylamino, N-Methyl-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Fluor, Chlor, Cyano, Carboxy, $C_1$-$C_4$-Alkoxy-carbonyl, $C_1$-$C_4$-Alkyl-carbonylamino, Dimethoxyphosphoryl oder Diethoxyphosphoryl steht und

$R^7$ für Formyl, Cyano, Carboxy, Hydroxymethyl, Carbamoyl, für jeweils gegebenenfalls durch Fluor, Chlor, Carboxy oder $C_1$-$C_4$-Alkoxy-carbonyl substituiertes $C_1$-$C_4$-Alkoxy-carbonyl, $C_5$-$C_6$-Cycloalkyloxy-carbonyl, $C_1$-$C_4$-Alkylthiocarbonyl, $C_1$-$C_4$-Alkylamino-carbonyl oder $C_5$-$C_6$-Cycloalkylamino-carbonyl, für Dimethylaminocarbonyl, für $C_1$-$C_4$-Alkylaminocarbonyl-$C_1$-$C_4$-alkoxy-carbonyl, für Dimethylaminocarbonyl-$C_1$-$C_4$-alkoxy-carbonyl, für N-Methyl-N-phenylaminocarbonyl-$C_1$-$C_4$-alkoxy-carbonyl, für jeweils gegebenenfalls durch Methyl und/oder Ethyl substituiertes Pyrrolidinyl-carbonyl, Piperidinyl-carbonyl, Morpholinyl-carbonyl oder Piperazinyl-carbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-N-phenylaminocarbonyl, Phenylhydrazinocarbonyl,

für Phthalimidoxycarbonyl oder zusammen mit $R^6$ für die Gruppierung -CO-O-$CH_2CH_2$- steht.

**3.** Substituierte Pyridine der Formel (I ) gemäß Ansprüch 1 in welcher

A für eine CH-Gruppierung steht,
Q für Sauerstoff steht,
$R^1$ für Methoxy steht,
$R^2$ für Methoxy steht,
$R^3$ für Wasserstoff oder Fluor steht,
$R^4$ für Wasserstoff steht und
Z die in Anspruch 2 angegebene Bedeutung hat.

**4.** Verfahren zur Herstellung von substituierten Pyridinen der Formel (I)

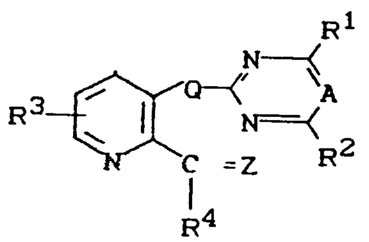

(I)

in welcher

A für Stickstoff oder eine C-X-Gruppierung steht, wobei X für Wasserstoff oder Halogen steht,

Q für Sauerstoff oder Schwefel steht,

$R^1$ und $R^2$ gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen,

$R^3$ für Wasserstoff, Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy, steht,

$R^4$ für Wasserstoff oder $C_1$-$C_4$-Alkyl steht und

Z für eine der nachstehenden Gruppierungen steht:
N-$R^5$ oder

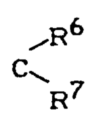

,

wobei

$R^5$ für Wasserstoff, Hydroxy, Amino oder für jeweils gegebenenfalls durch Halogen substituiertes $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkyl-carbonylamino, $C_1$-$C_6$-Alkoxy-carbonylamino, $C_1$-$C_6$-Alkyl-sulfonylamino, oder für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenoxy, Phenyl-$C_1$-$C_4$-alkoxy, Phenylamino, Phenyl-$C_1$-$C_4$-alkylamino, N-($C_1$-$C_4$-alkyl)-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Halogen, Cyano, Carboxy, $C_1$-$C_6$-Alkoxy-carbonyl, $C_1$-$C_6$-Alkyl-carbonylamino oder Di-($C_1$-$C_4$-alkoxy)-phosphoryl steht und

$R^7$ für Formyl, Cyano, Carboxy, Hydroxymethyl, Carbamoyl oder für jeweils gegebenenfalls durch Halogen, Carboxy oder $C_1$-$C_4$-Alkoxy-carbonyl substituiertes $C_1$-$C_6$-Alkoxy-

carbonyl, $C_5$-$C_6$-Cycloalkyloxy-carbonyl, $C_1$-$C_6$-Alkylthio-carbonyl, $C_1$-$C_6$-Alkylamino-carbonyl oder $C_5$-$C_6$-Cycloalkylamino-carbonyl, für Di-($C_1$-$C_2$-alkyl)-amino-carbonyl, für $C_1$-$C_4$-Alkyl-amino-carbonyl-$C_1$-$C_4$-alkoxy-carbonyl, für Di-($C_1$-$C_2$-alkyl)-amino-carbonyl-$C_1$-$C_4$-alkoxy-carbonyl, für Phenylaminocarbonyl-$C_1$-$C_4$-alkoxy-carbonyl, für N-Methyl-N-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthio-carbonyl, Phenylaminocarbonyl, Phenyl-$C_1$-$C_4$-alkylamino-carbonyl, N-($C_1$-$C_4$-Alkyl)-N-phenylamino-carbonyl oder Phenylhydrazinocarbonyl oder $C_1$-$C_4$-Alkylhydrazinocarbonyl, für Phthalimidoxycarbonyl oder zusammen mit $R^6$ für die Gruppierung $-CO-O-(CH_2)_n$-steht, wobei

n für die Zahlen 1 bis 4 steht,

dadurch gekennzeichnet, daß man substituierte Pyridine der allgemeinen Formel (Ia)

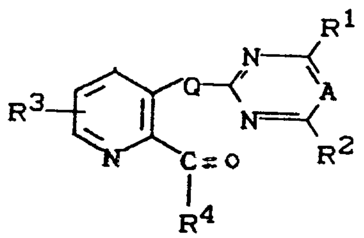

(Ia)

in welcher

A, Q, $R^1$, $R^2$, $R^3$ und $R^4$ die oben angegebene Bedeutung haben,

mit Amino- oder Methylen-Verbindungen der allgemeinen Formel (III)

$H_2Z$ (III)

in welcher

Z die oben angegebene Bedeutung hat,

gegebenenfalls in Gegenwart eines Reaktionshilfsmittels und gegebenenfalls in Gegenwart eines Verdünnungsmittels umsetzt.

**5.** Herbizide Mittel, gekennzeichnet durch ein Gehalt an mindestens einem substituierten Pyridin-Derivat der Formel (I) gemäß den Ansprüchen 1 bis 3.

**6.** Verfahren zur Bekämpfung von unerwünschten Pflanzen, dadurch gekennzeichnet, daß man substituierte Pyridine der Formel (I) gemäß den Ansprüchen 1 bis 3 auf unerwünschte Pflanzen und/oder ihren Lebensraum einwirken läßt.

**7.** Verwendung von substituierten Pyridinen der Formel (I) gemäß den Ansprüchen 1 bis 3 zur Bekämpfung von unerwünschten Pflanzen.

**8.** Verfahren zur Herstellung von herbiziden Mitteln, dadurch gekennzeichnet, daß man substituierte Pyridine der Formel (I) gemäß den Ansprüchen 1 bis 3 mit Streckmitteln und/oder oberflächenaktiven Substanzen mischt.

**9.** Hydroxyalkylpyridine der allgemeinen Formel (II)

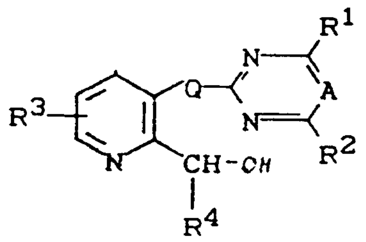

(II)

in welcher

| | |
|---|---|
| A | für Stickstoff oder eine C-X-Gruppierung steht, wobei X für Wasserstoff oder Halogen steht, |
| Q | für Sauerstoff oder Schwefel steht, |
| $R^1$ und $R^2$ | gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen, |
| $R^3$ | für Wasserstoff, Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy, steht, |
| $R^4$ | für Wasserstoff oder $C_1$-$C_4$-Alkyl steht. |

**Patentansprüche für folgenden Vertragsstaat : ES**

**1.** Verfahren zur Herstllung substituierter Pyridine der allgemeinen Formel (I)

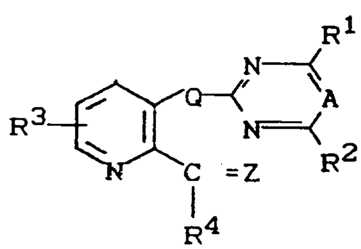

(I)

in welcher

| | |
|---|---|
| A | für Stickstoff oder eine C-X-Gruppierung steht, wobei X für Wasserstoff oder Halogen steht, |
| Q | für Sauerstoff oder Schwefel steht, |
| $R^1$ und $R^2$ | gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen, |
| $R^3$ | für Wasserstoff, , Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy, steht, |
| $R^4$ | für Wasserstoff oder $C_1$-$C_4$-Alkyl steht und |
| Z | für eine der nachstehenden Gruppierungen steht: N-$R^5$ oder |

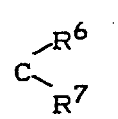 ,

wobei

| | |
|---|---|
| $R^5$ | für Wasserstoff, Hydroxy, Amino oder für jeweils gegebenenfalls durch Halogen |

substituiertes $C_1$-$C_6$-Alkyl, $C_3$-$C_6$-Alkenyl, $C_3$-$C_6$-Alkinyl, $C_1$-$C_6$-Alkoxy, $C_3$-$C_6$-Alkenyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkyl-carbonylamino, $C_1$-$C_6$-Alkoxy-carbonylamino, $C_1$-$C_6$-Alkyl-sulfonylamino, oder für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenyl, Phenyl-$C_1$-$C_4$-alkyl, Phenoxy, Phenyl-$C_1$-$C_4$-alkoxy, Phenylamino, Phenyl-$C_1$-$C_4$-alkylamino, N-($C_1$-$C_4$-alkyl)-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Halogen, Cyano, Carboxy, $C_1$-$C_6$-Alkoxy-carbonyl, $C_1$-$C_6$-Alkyl-carbonylamino oder Di-($C_1$-$C_4$-alkoxy)phosphoryl steht und

$R^7$ für Formyl, Cyano, Carboxy, Hydroxymethyl, Carbamoyl oder für jeweils gegebenenfalls durch Halogen, Carboxy oder $C_1$-$C_4$-Alkoxy-carbonyl substituiertes $C_1$-$C_6$-Alkoxycarbonyl, $C_5$-$C_6$-Cycloalkyloxy-carbonyl, $C_1$-$C_6$-Alkylthio-carbonyl, $C_1$-$C_6$-Alkylaminocarbonyl oder $C_5$-$C_6$-Cycloalkylamino-carbonyl, für Di-($C_1$-$C_2$-alkyl)-amino-carbonyl, für $C_1$-$C_4$-Alkylamino-carbonyl-$C_1$-$C_4$-alkoxy-carbonyl, für Di-($C_1$-$C_2$-alkyl)-amino-carbonyl-$C_1$-$C_4$-alkoxy-carbonyl, für Phenylaminocarbonyl$C_1$-$C_4$-alkoxy-carbonyl, für M-Methyl-N-phenylaminocarbonyl-$C_1$-$C_4$-alkoxy-carbonyl, für jeweils gegebenenfalls durch Methyl und/oder Ethyl substituiertes Pyrrolidinylcarbonyl, Piperidinylcarbonyl, Morpholinylcarbonyl oder Piperazinylcarbonyl, für jeweils gegebenenfalls durch Nitro, Amino, Cyano, Carboxy, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituiertes Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthio-carbonyl, Phenylaminocarbonyl, Phenyl-$C_1$-$C_4$-alkylamino-carbonyl, N-($C_1$-$C_4$-Alkyl)-N-phenylamino-carbonyl oder Phenylhydrazinocarbonyl oder $C_1$-$C_4$-Alkylhydrazinocarbonyl, für Phthalimidoxycarbonyl oder zusammen mit $R^6$ für die Gruppierung $-CO-O-(CH_2)_n-$steht, wobei

n für die Zahlen 1 bis 4 steht,

dadurch gekennzeichnet, daß man substituierte Pyridine der allgemeinen Formel (Ia)

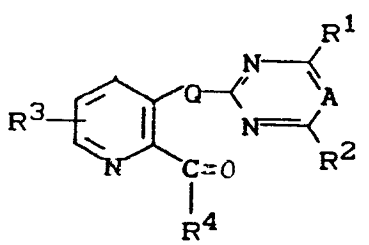

(Ia)

in welcher

A, Q, $R^1$, $R^2$, $R^3$ und $R^4$ die oben angegebene Bedeutung haben,

mit Amino- oder Methylen-Verbindungen der allgemeinen Formel (III)

$H_2Z$ (III)

in welcher

Z die oben angegebene Bedeutung hat,

gegebenenfalls in Gegenwart eines Reaktionshilfsmittels und gegebenenfalls in Gegenwart eines Verdünnungsmittels umsetzt.

2. Herbizide Mittel, gekennzeichnet durch ein Gehalt an mindestens einem substituierten Pyridin-Derivat der Formel (I) gemäß dem Anspruch 1.

3. Verfahren zur Bekämpfung von unerwünschten Pflanzen, dadurch gekennzeichnet, daß man substituierte Pyridine der Formel (I) gemäß Anspruch 1 auf unerwünschte Pflanzen und/oder ihren Lebensraum

einwirken läßt.

4. Verwendung von substituierten Pyridinen der Formel (I) gemäß Anspruch 1 zur Bekämpfung von unerwünschten Pflanzen.

5. Verfahren zur Herstellung von herbiziden Mitteln, dadurch gekennzeichnet, daß man substituierte Pyridine der Formel (I) gemäß dem Anspruch 1 mit Streckmitteln und/oder oberflächenaktiven Substanzen mischt.

6. Verfahren zur Herstellung von Hydroxyalkylpyridinen der allgemeinen Formel (II)

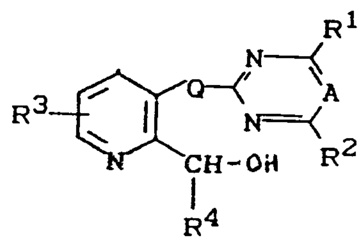

(II)

in welcher

A für Stickstoff oder eine C-X-Gruppierung steht, wobei X für Wasserstoff oder Halogen steht,

Q für Sauerstoff oder Schwefel steht,

$R^1$ und $R^2$ gleich oder verschieden sind und für Wasserstoff, Halogen, $C_1$-$C_4$-Alkyl, $C_1$-$C_4$-Halogenalkyl, $C_1$-$C_2$-Alkoxy-$C_1$-$C_2$-alkyl, $C_1$-$C_4$-Alkoxy, $C_1$-$C_4$-Halogenalkoxy, $C_1$-$C_4$-Alkylthio, $C_1$-$C_4$-Alkylamino oder Di-($C_1$-$C_2$-alkyl)-amino stehen,

$R^3$ für Wasserstoff, , Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy, steht,

$R^4$ für Wasserstoff oder $C_1$-$C_4$-Alkyl steht.

7. Verfahren zur Herstellung substituierter Pyridine der Formel (I) gemäß Anspruch 1, in welcher

A für Stickstoff oder eine CH-Gruppierung steht,

Q für Sauerstoff steht,

$R^1$ für Wasserstoff, Chlor, Methyl, Ethyl, Trifluormethyl, Methoxymethyl, Methoxy, Ethoxy, Difluormethoxy, Methylthio, Methylamino, Ethylamino oder Dimethylamino steht,

$R^2$ für Wasserstoff, Chlor, Methyl, Ethyl, Trifluormethyl, Methoxy, Ethoxy, Difluormethoxy, Methylthio, Methylamino, Ethylamino oder Dimethylamino steht,

$R^3$ für Wasserstoff, Hydroxy, Fluor, Chlor, Brom, Methyl, Ethyl, Trifluormethyl, Methoxy, Difluormethoxy, Trifluormethoxy, steht,

$R^4$ für Wasserstoff oder Methyl steht und

Z für eine der nachstehenden Gruppierungen steht:

N-$R^5$ oder

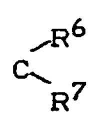

,

wobei

$R^5$ für Wasserstoff, Hydroxy, Amino, für Methyl, Ethyl, n-Propyl, Isopropyl, n-Butyl, Isobutyl, sec-Butyl, tert-Butyl, Allyl, Propargyl, Methoxy, Ethoxy, n-Propoxy, Isopropoxy, n-Butoxy, Isobutoxy, sec-Butoxy, Allyloxy, Methoxycarbonylmethoxy, Ethoxycarbonylmethoxy, Methoxycarbonylethoxy, Ethoxycarbonylethoxy, Methylamino, Ethylamino, n-Propylamino, Isopropylamino, n-Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenyl, Benzyl, Phenoxy, Benzyloxy, Phenylamino, Benzylamino, N-Methyl-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^6$ für Wasserstoff, Fluor, Chlor, Cyano, Carboxy, $C_1$-$C_4$-Alkoxy-carbonyl, $C_1$-$C_4$-Alkyl-carbonylamino, Dimethoxyphosphoryl oder Diethoxyphosphoryl steht und

$R^7$ für Formyl, Cyano, Carboxy, Hydroxymethyl, Carbamoyl, für jeweils gegebenenfalls durch Fluor, Chlor, Carboxy oder $C_1$-$C_4$-Alkoxy-carbonyl substituiertes $C_1$-$C_4$-Alkoxy-carbonyl, $C_5$-$C_6$-Cycloalkyloxy-carbonyl, $C_1$-$C_4$-Alkylthiocarbonyl, $C_1$-$C_4$-Alkylamino-carbonyl oder $C_5$-$C_6$-Cycloalkylamino-carbonyl, für Dimethylaminocarbonyl, für $C_1$-$C_4$-Alkylaminocarbonyl-$C_1$-$C_4$-alkoxy-carbonyl, für Dimethylaminocarbonyl-$C_1$-$C_4$-alkoxy-carbonyl, für M-Methyl-M-phenylaminocarbonyl-$C_1$-$C_4$-alkoxy-carbonyl, für jeweils gegebenenfalls durch Methyl und/oder Ethyl substituiertes Pyrrolidinyl-carbonyl, Piperidinyl-carbonyl, Morpholinyl-carbonyl oder Piperazinyl-carbonyl, für jeweils gegebenenfalls durch Nitro, Cyano, Fluor, Chlor, Brom, Methyl, Trifluormethyl, Methoxy, Trifluormethoxy, Methylthio oder Trifluormethylthio substituiertes Phenoxycarbonyl, Benzyloxycarbonyl, Phenylthiocarbonyl, Benzylthiocarbonyl, Phenylaminocarbonyl, Benzylaminocarbonyl, N-Methyl-N-phenylaminocarbonyl, Phenylhydrazinocarbonyl, für Phthalimidoxycarbonyl oder zusammen mit $R^6$ für die Gruppierung $-CO-O-CH_2CH_2-$ steht.

**8.** Verfahren zur Herstellung substituierter Pyridine der Formel (I) gemäß Anspruch 1 in welcher

A für eine CH-Gruppierung steht,
Q für Sauerstoff steht,
$R^1$ für Methoxy steht,
$R^2$ für Methoxy steht,
$R^3$ für Wasserstoff oder Fluor steht,
$R^4$ für Wasserstoff steht und
Z die in Anspruch 7 angegebene Bedeutung hat.

## Claims

**Claims for the following Contracting States: BE, CH, DE, FR, GB, IT, LI, NL**

**1.** Substituted pyridines of the general formula (I)

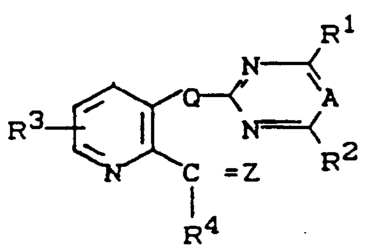

(I)

in which

A represents nitrogen or a C-X grouping, where X represents hydrogen or halogen,
Q represents oxygen or sulphur,
$R^1$ and $R^2$ are identical or different and represent hydrogen, halogen, $C_1$-$C_4$-alkyl, $C_1$-$C_4$-halogenoalkyl, $C_1$-$C_2$-alkoxy-$C_1$-$C_2$-alkyl, $C_1$-$C_4$-alkoxy, $C_1$-$C_4$-halogenoalkoxy, $C_1$-$C_4$-alkylthio, $C_1$-$C_4$-alkylamino or di-($C_1$-$C_2$-alkyl)-amino,
$R^3$ represents hydrogen, hydroxyl, halogen, $C_1$-$C_4$-alkyl, $C_1$-$C_4$-halogenoalkyl, $C_1$-$C_4$-alkoxy or $C_1$-$C_4$-halogenoalkoxy,
$R^4$ represents hydrogen or $C_1$-$C_4$-alkyl, and
Z represents one of the following groupings:
N-$R^5$ or

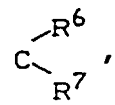

where

$R^5$ represents hydrogen, hydroxyl or amino, or represents $C_1$-$C_6$-alkyl, $C_3$-$C_6$-alkenyl, $C_3$-$C_6$-alkinyl, $C_1$-$C_6$-alkoxy, $C_3$-$C_6$-alkenyloxy, $C_1$-$C_4$-alkoxy-carbonyl-$C_1$-$C_2$-alkoxy, $C_1$-$C_6$-alkylamino, di-($C_1$-$C_2$-alkyl)-amino, $C_1$-$C_6$-alkyl-carbonylamino, $C_1$-$C_6$-alkoxy-carbonylamino or $C_1$-$C_6$-alkyl-sulphonylamino, each of which is optionally substituted by halogen, or represents phenyl, phenyl$C_1$-$C_4$-alkyl, phenoxy, phenyl-$C_1$-$C_4$-alkoxy, phenylamino, phenyl-$C_1$-$C_4$-alkylamino, N-($C_1$-$C_4$-alkyl)-N-phenylamino, pyridylamino, pyrimidylamino, pyridylcarbonylamino, phenylcarbonylamino, furylcarbonylamino, thienylcarbonylamino or phenylsulphonylamino each of which is optionally substituted by nitro, amino, cyano, carboxyl, halogen, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkylthio, $C_1$-$C_2$-halogenoalkylthio, $C_1$-$C_4$-alkoxycarbonyl and/or di-($C_1$-$C_2$-alkyl)-amino,

$R^6$ represents hydrogen, halogen, cyano, carboxyl, $C_1$-$C_6$-alkoxy-carbonyl, $C_1$-$C_6$-alkyl-carbonylamino or di-($C_1$-$C_4$-alkoxy)-phosphoryl, and

$R^7$ represents formyl, cyano, carboxyl, hydroxymethyl or carbamoyl, or represents $C_1$-$C_6$-alkoxy-carbonyl, $C_5$-$C_6$-cycloalkyloxy-carbonyl, $C_1$-$C_6$-alkylthio-carbonyl, $C_1$-$C_6$-alkylaminocarbonyl or $C_5$-$C_6$-cycloalkylamino-carbonyl, each of which is optionally substituted by halogen, carboxyl or $C_1$-$C_4$-alkoxy-carbonyl, represents di-($C_1$-$C_2$-alkyl)-amino-carbonyl, represents $C_1$-$C_4$-alkyl-amino-carbonyl-$C_1$-$C_4$-alkoxy-carbonyl, represents di-($C_1$-$C_2$-alkyl)-amino-carbonyl-$C_1$-$C_4$-alkoxy-carbonyl, represents phenylaminocarbonyl-$C_1$-$C_4$-alkoxy-carbonyl, represents N-methyl-N-phenylaminocarbonyl-$C_1$-$C_4$-alkoxy-carbonyl, represents pyrrolidinylcarbonyl, piperidinylcarbonyl, morpholinylcarbonyl or piperazinylcarbonyl, each of which is optionally substituted by methyl and/or ethyl, represents phenoxycarbonyl, phenyl-$C_1$-$C_4$-alkoxycarbonyl, furylmethoxycarbonyl, thienylmethoxycarbonyl, phenylthiocarbonyl, phenyl-$C_1$-$C_4$-alkylthiocarbonyl, phenylaminocarbonyl, phenyl-$C_1$-$C_4$-alkylamino-carbonyl, N-($C_1$-$C_4$-alkyl)-N-phenylamino-carbonyl or phenylhydrazinocarbonyl or $C_1$-$C_4$-alkylhydrazinocarbonyl, each of which is optionally substituted by nitro, amino, cyano, carboxyl, halogen, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkylthio, $C_1$-$C_2$-halogenoalkylthio, $C_1$-$C_4$-alkoxy-carbonyl and/or di-($C_1$-$C_2$-alkyl)-amino, represents phthalimidoxycarbonyl, or, together with $R^6$, represents the grouping $-CO-O-(CH_2)_n-$, where

n represents the numbers from 1 to 4.

**2.** Substituted pyridines of the formula (I) according to Claim 1, in which

A represents nitrogen or a CH grouping,

Q represents oxygen,

$R^1$ represents hydrogen, chlorine, methyl, ethyl, trifluoromethyl, methoxymethyl, methoxy, ethoxy, difluoromethoxy, methylthio, methylamino, ethylamino or dimethylamino,

$R^2$ represents hydrogen, chlorine, methyl, ethyl, trifluoromethyl, methoxy, ethoxy, difluoromethoxy, methylthio, methylamino, ethylamino or dimethylamino,

$R^3$ represents hydrogen, hydroxyl, fluorine, chlorine, bromine, methyl, ethyl, trifluoromethyl, methoxy, difluoromethoxy or trifluoromethoxy,

$R^4$ represents hydrogen or methyl, and

Z represents one of the following groupings:

N-$R^5$ or

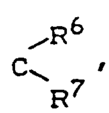

where

$R^5$ represents hydrogen, hydroxyl or amino, represents methyl, ethyl, n-propyl, isopropyl, n-butyl, isobutyl, sec-butyl, tert-butyl, allyl, propargyl, methoxy, ethoxy, n-propoxy, isopropoxy, n-butoxy, isobutoxy, sec-butoxy, allyloxy, methoxycarbonylmethoxy, ethoxycarbonylmethoxy, methoxycarbonylethoxy, ethoxycarbonylethoxy, methylamino, ethylamino, n-propylamino, isopropylamino, n-butylamino, isobutylamino, sec-butylamino, tert-butylamino, dimethylamino, acetylamino, propionylamino, methoxycarbonylamino, ethoxycarbonylamino, methylsulphonylamino or ethylsulphonylamino, represents phenyl, benzyl, phenoxy, benzyloxy, phenylamino, benzylamino, N-methyl-N-phenylamino, pyridylamino, pyrimidylamino, pyridylcarbonylamino, phenylcarbonylamino, furylcarbonylamino, thienylcarbonylamino or phenylsulphonylamino, each of which is optionally substituted by nitro, cyano, fluorine, chlorine, bromine, methyl, trifluoromethyl, methoxy, trifluoromethoxy, methylthio or trifluoromethylthio,

$R^6$ represents hydrogen, fluorine, chlorine, cyano, carboxyl, $C_1$-$C_4$-alkoxy-carbonyl, $C_1$-$C_4$-alkylcarbonylamino, dimethoxyphosphoryl or diethoxyphosphoryl, and

$R^7$ represents formyl, cyano, carboxyl, hydroxymethyl or carbamoyl, represents $C_1$-$C_4$-alkoxycarbonyl, $C_5$-$C_6$-cycloalkyloxy-carbonyl, $C_1$-$C_4$-alkylthiocarbonyl, $C_1$-$C_4$-alkylaminocarbonyl or $C_5$-$C_6$-cycloalkylamino-carbonyl, each of which is optionally substituted by fluorine, chlorine, carboxyl or $C_1$-$C_4$-alkoxy-carbonyl, represents dimethylaminocarbonyl, represents $C_1$-$C_4$-alkylaminocarbonyl-$C_1$-$C_4$-alkoxy-carbonyl, represents dimethylaminocarbonyl-$C_1$-$C_4$-alkoxy-carbonyl, represents N-methyl-N-phenylaminocarbonyl-$C_1$-$C_4$-alkoxy-carbonyl, represents pyrrolidinyl-carbonyl, piperidinyl-carbonyl, morpholinyl-carbonyl or piperazinyl-carbonyl, each of which is optionally substituted by methyl and/or ethyl, represents phenoxycarbonyl, benzyloxycarbonyl, phenylthiocarbonyl, benzylthiocarbonyl, phenylaminocarbonyl, benzylaminocarbonyl, N-methyl-N-phenylaminocarbonyl or phenylhydrazinocarbonyl, each of which is optionally substituted by nitro, cyano, fluorine, chlorine, bromine, methyl, trifluoromethyl, methoxy, trifluoromethoxy, methylthio or trifluoromethylthio, represents phthalimidoxycarbonyl, or, together with $R^6$, represents the grouping $-CO-O-CH_2CH_2-$.

**3.** Substituted pyridines of the formula (I) according to Claim 1 in which

A represents a CH grouping,
Q represents oxygen,
$R^1$ represents methoxy,
$R^2$ represents methoxy,
$R^3$ represents hydrogen or fluorine,
$R^4$ represents hydrogen, and
Z has the meaning given in Claim 2.

**4.** Process for preparing substituted pyridines of the formula (I)

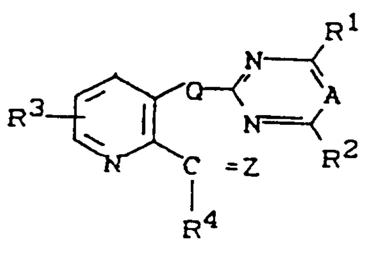

(I)

in which

A represents nitrogen or a C-X grouping, where X represents hydrogen or halogen,
Q represents oxygen or sulphur,
$R^1$ and $R^2$ are identical or different and represent hydrogen, halogen, $C_1$-$C_4$-alkyl, $C_1$-$C_4$-halogenoalkyl, $C_1$-$C_2$-alkoxy-$C_1$-$C_2$-alkyl, $C_1$-$C_4$-alkoxy, $C_1$-$C_4$-halogenoalkoxy, $C_1$-$C_4$-alkylthio, $C_1$-$C_4$-alkylamino or di-($C_1$-$C_2$-alkyl)-amino,
$R^3$ represents hydrogen, hydroxyl, halogen, $C_1$-$C_4$-alkyl, $C_1$-$C_4$-halogenoalkyl, $C_1$-$C_4$-alkoxy or $C_1$-$C_4$-halogenoalkoxy,

$R^4$ represents hydrogen or $C_1$-$C_4$-alkyl, and

Z represents one of the following groupings:

N-$R^5$ or

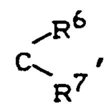

where

$R^5$ represents hydrogen, hydroxyl or amino, or represents $C_1$-$C_6$-alkyl, $C_3$-$C_6$-alkenyl, $C_3$-$C_6$-alkinyl, $C_1$-$C_6$-alkoxy, $C_3$-$C_6$-alkenyloxy, $C_1$-$C_4$-alkoxy-carbonyl-$C_1$-$C_2$-alkoxy, $C_1$-$C_6$-alkylamino, di-($C_1$-$C_2$-alkyl)-amino, $C_1$-$C_6$-alkyl-carbonylamino, $C_1$-$C_6$-alkoxy-carbonylamino or $C_1$-$C_6$-alkyl-sulphonylamino, each of which is optionally substituted by halogen, or represents phenyl, phenyl-$C_1$-$C_4$-alkyl, phenoxy, phenyl-$C_1$-$C_4$-alkoxy, phenylamino, phenyl-$C_1$-$C_4$-alkylamino, N-($C_1$-$C_4$-alkyl)-N-phenylamino, pyridylamino, pyrimidylamino, pyridylcarbonylamino, phenylcarbonylamino, furylcarbonylamino, thienylcarbonylamino or phenylsulphonylamino each of which is optionally substituted by nitro, amino, cyano, carboxyl, halogen, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkylthio, $C_1$-$C_2$-halogenoalkylthio, $C_1$-$C_4$-alkoxycarbonyl and/or di-($C_1$-$C_2$-alkyl)-amino,

$R^6$ represents hydrogen, halogen, cyano, carboxyl, $C_1$-$C_6$-alkoxy-carbonyl, $C_1$-$C_6$-alkyl-carbonylamino or di-($C_1$-$C_4$-alkoxy)-phosphoryl, and

$R^7$ represents formyl, cyano, carboxyl, hydroxymethyl or carbamoyl, or represents $C_1$-$C_6$-alkoxy-carbonyl, $C_5$-$C_6$-cycloalkyloxy-carbonyl, $C_1$-$C_6$-alkylthio-carbonyl, $C_1$-$C_6$-alkylaminocarbonyl or $C_5$-$C_6$-cycloalkylamino-carbonyl, each of which is optionally substituted by halogen, carboxyl or $C_1$-$C_4$-alkoxy-carbonyl, represents di-($C_1$-$C_2$-alkyl)-amino-carbonyl, represents $C_1$-$C_4$-alkyl-amino-carbonyl-$C_1$-$C_4$-alkoxy-carbonyl, represents di-($C_1$-$C_2$-alkyl)-amino-carbonyl-$C_1$-$C_4$-alkoxy-carbonyl, represents phenylaminocarbonyl-$C_1$-$C_4$-alkoxy-carbonyl, represents N-methyl-N-phenylaminocarbonyl-$C_1$-$C_4$-alkoxy-carbonyl, represents pyrrolidinylcarbonyl, piperidinylcarbonyl, morpholinylcarbonyl or piperazinylcarbonyl, each of which is optionally substituted by methyl and/or ethyl, represents phenoxycarbonyl, phenyl-$C_1$-$C_4$-alkoxycarbonyl, furylmethoxycarbonyl, thienylmethoxycarbonyl, phenylthiocarbonyl, phenyl-$C_1$-$C_4$-alkylthiocarbonyl, phenylaminocarbonyl, phenyl-$C_1$-$C_4$-alkylamino-carbonyl, N-($C_1$-$C_4$-alkyl)-N-phenylamino-carbonyl or phenylhydrazinocarbonyl or $C_1$-$C_4$-alkylhydrazinocarbonyl, each of which is optionally substituted by nitro, amino, cyano, carboxyl, halogen, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkylthio, $C_1$-$C_2$-halogenoalkylthio, $C_1$-$C_4$-alkoxy-carbonyl and/or di-($C_1$-$C_2$-alkyl)-amino, represents phthalimidoxycarbonyl, or, together with $R^6$, represents the grouping $-CO-O-(CH_2)_n-$, where

n represents the numbers from 1 to 4,

characterized in that,

substituted pyridines of the general formula (Ia)

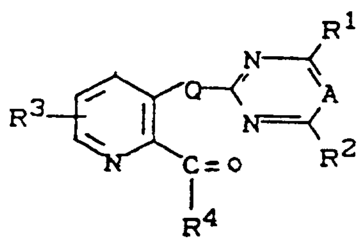

(Ia)

in which

A, Q, $R^1$, $R^2$, $R^3$ and $R^4$ have the abovementioned meaning,

are reacted with amino or methylene compounds of the general formula (III)

$H_2Z$ (III)

in which

Z has the abovementioned meaning,

optionally in the presence of a reaction auxiliary and optionally in the presence of a diluent.

**5.** Herbicidal agent, characterized by a content of at least one substituted pyridine derivative of the formula (I) according to Claims 1 to 3.

**6.** Process for controlling unwanted plants, characterized in that substituted pyridines of the formula (I) according to Claims 1 to 3 are allowed to act on unwanted plants and/or their habitat.

**7.** Use of substituted pyridines of the formula (I) according to Claims 1 to 3 for controlling unwanted plants.

**8.** Process for preparing herbicidal agents, characterized in that substituted pyridines of the formula (I) according to Claims 1 to 3 are mixed with extenders and/or surface-active substances.

**9.** Hydroxyalkylpyridines of the general formula (II)

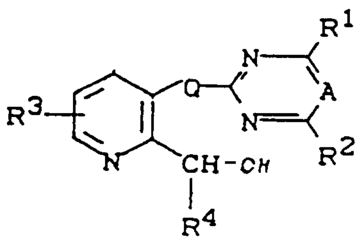

(II)

in which

| | |
|---|---|
| A | represents nitrogen or a C-X grouping, where X represents hydrogen or halogen, |
| Q | represents oxygen or sulphur, |
| $R^1$ and $R^2$ | are identical or different, and represent hydrogen, halogen, $C_1$-$C_4$-alkyl, $C_1$-$C_4$-halogenoalkyl, $C_1$-$C_2$-alkoxy-$C_1$-$C_2$-alkyl, $C_1$-$C_4$-alkoxy, $C_1$-$C_4$-halogenoalkoxy, $C_1$-$C_4$-alkylthio, $C_1$-$C_4$-alkylamino or di-($C_1$-$C_2$-alkyl)-amino, |
| $R^3$ | represents hydrogen, hydroxyl, halogen, $C_1$-$C_4$-alkyl, $C_1$-$C_4$-halogenoalkyl, $C_1$-$C_4$-alkoxy or $C_1$-$C_4$-halogenoalkoxy, |
| $R^4$ | represents hydrogen or $C_1$-$C_4$-alkyl. |

**Claims for the following Contracting State : ES**

**1.** Process for preparing substituted pyridines of the general formula (I)

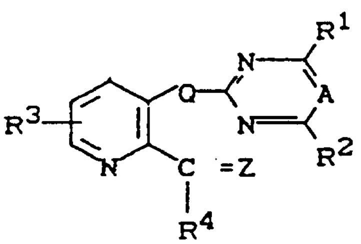

(I)

in which

| | |
|---|---|
| A | represents nitrogen or a C-X grouping, where X represents hydrogen or halogen, |
| Q | represents oxygen or sulphur, |
| $R^1$ and $R^2$ | are identical or different and represent hydrogen, halogen, $C_1$-$C_4$-alkyl, $C_1$-$C_4$- |

halogenoalkyl, $C_1$-$C_2$-alkoxy-$C_1$-$C_2$-alkyl, $C_1$-$C_4$-alkoxy, $C_1$-$C_4$-halogenoalkoxy, $C_1$-$C_4$-alkylthio, $C_1$-$C_4$-alkylamino or di-($C_1$-$C_2$-alkyl)-amino,

$R^3$ represents hydrogen, hydroxyl, halogen, $C_1$-$C_4$-alkyl, $C_1$-$C_4$-halogenoalkyl, $C_1$-$C_4$-alkoxy or $C_1$-$C_4$-halogenoalkoxy,

$R^4$ represents hydrogen or $C_1$-$C_4$-alkyl, and

Z represents one of the following groupings:
N-$R^5$ or

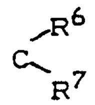 ,

where

$R^5$ represents hydrogen, hydroxyl or amino, or represents $C_1$-$C_6$-alkyl, $C_3$-$C_6$-alkenyl, $C_3$-$C_6$-alkinyl, $C_1$-$C_6$-alkoxy, $C_3$-$C_6$-alkenyloxy, $C_1$-$C_4$-alkoxy-carbonyl-$C_1$-$C_2$-alkoxy, $C_1$-$C_6$-alkylamino, di-($C_1$-$C_2$-alkyl)-amino, $C_1$-$C_6$-alkyl-carbonylamino, $C_1$-$C_6$-alkoxy-carbonylamino or $C_1$-$C_6$-alkyl-sulphonylamino, each of which is optionally substituted by halogen, or represents phenyl, phenyl-$C_1$-$C_4$-alkyl, phenoxy, phenyl-$C_1$-$C_4$-alkoxy, phenylamino, phenyl-$C_1$-$C_4$-alkylamino, N-($C_1$-$C_4$-alkyl)-N-phenylamino, pyridylamino, pyrimidylamino, pyridylcarbonylamino, phenylcarbonylamino, furylcarbonylamino, thienylcarbonylamino or phenylsulphonylamino each of which is optionally substituted by nitro, amino, cyano, carboxyl, halogen, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkylthio, $C_1$-$C_2$-halogenoalkylthio, $C_1$-$C_4$-alkoxycarbonyl and/or di-($C_1$-$C_2$-alkyl)-amino,

$R^6$ represents hydrogen, halogen, cyano, carboxyl, $C_1$-$C_6$-alkoxy-carbonyl, $C_1$-$C_6$-alkyl-carbonylamino or di-($C_1$-$C_4$-alkoxy)-phosphoryl, and

$R^7$ represents formyl, cyano, carboxyl, hydroxymethyl or carbamoyl, or represents $C_1$-$C_6$-alkoxycarbonyl, $C_5$-$C_6$-cycloalkyloxy-carbonyl, $C_1$-$C_6$-alkylthio-carbonyl, $C_1$-$C_6$-alkylamino-carbonyl or $C_5$-$C_6$-cycloalkylamino-carbonyl, each of which is optionally substituted by halogen, carboxyl or $C_1$-$C_4$-alkoxy-carbonyl, represents di-($C_1$-$C_2$-alkyl)-amino-carbonyl, represents $C_1$-$C_4$-alkyl-amino-carbonyl-$C_1$-$C_4$-alkoxy-carbonyl, represents di-($C_1$-$C_2$-alkyl)-amino-carbonyl-$C_1$-$C_4$-alkoxy-carbonyl, represents phenylaminocarbonyl-$C_1$-$C_4$-alkoxy-carbonyl, represents N-methyl-N-phenylaminocarbonyl-$C_1$-$C_4$-alkoxy-carbonyl, represents pyrrolidinylcarbonyl, piperidinylcarbonyl, morpholinylcarbonyl or piperazinylcarbonyl, each of which is optionally substituted by methyl and/or ethyl, represents phenoxycarbonyl, phenyl-$C_1$-$C_4$-alkoxycarbonyl, furylmethoxycarbonyl, thienylmethoxycarbonyl, phenylthiocarbonyl, phenyl-$C_1$-$C_4$-alkylthiocarbonyl, phenylaminocarbonyl, phenyl-$C_1$-$C_4$-alkylamino-carbonyl, N-($C_1$-$C_4$-alkyl)-N-phenylamino-carbonyl or phenylhydrazinocarbonyl or $C_1$-$C_4$-alkylhydrazino-carbonyl, each of which is optionally substituted by nitro, amino, cyano, carboxyl, halogen, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkyl-thio, $C_1$-$C_2$-halogenoalkylthio, $C_1$-$C_4$-alkoxy-carbonyl and/or di-($C_1$-$C_2$-alkyl)-amino, represents phthalimidoxycarbonyl, or, together with $R^6$, represents the grouping $-CO-O-(CH_2)_n-$, where

n represents the numbers from 1 to 4,

characterized in that,
substituted pyridines of the general formula (Ia)

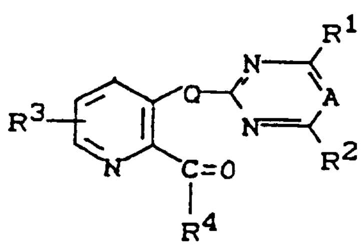

(Ia)

in which

A, Q, $R^1$, $R^2$, $R^3$ and $R^4$ have the abovementioned meaning,

are reacted with amino or methylene compounds of the general formula (III)

$H_2Z$ (III)

in which

Z has the abovementioned meaning,

optionally in the presence of a reaction auxiliary and optionally in the presence of a diluent.

**2.** Herbicidal agent, characterized by a content of at least one substituted pyridine derivative of the formula (I) according to Claim 1.

**3.** Process for controlling unwanted plants, characterized in that substituted pyridines of the formula (I) according to Claim 1 are allowed to act on unwanted plants and/or their habitat.

**4.** Use of substituted pyridines of the formula (I) according to Claim 1 for controlling unwanted plants.

**5.** Process for preparing herbicidal agents, characterized in that substituted pyridines of the formula (I) according to Claim 1 are mixed with extenders and/or surface-active substances.

**6.** Process for preparing hydroxyalkylpyridines of the general formula (II)

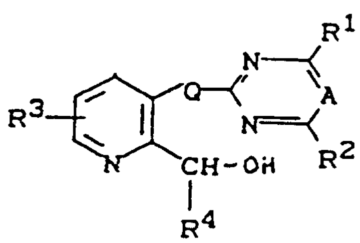

(II)

in which

A represents nitrogen or a C-X grouping, where X represents hydrogen or halogen,

Q represents oxygen or sulphur,

$R^1$ and $R^2$ are identical or different, and represent hydrogen, halogen, $C_1$-$C_4$-alkyl, $C_1$-$C_4$-halogenoalkyl, $C_1$-$C_2$-alkoxy-$C_1$-$C_2$-alkyl, $C_1$-$C_4$-alkoxy, $C_1$-$C_4$-halogenoalkoxy, $C_1$-$C_4$-alkylthio, $C_1$-$C_4$-alkylamino or di-($C_1$-$C_2$-alkyl)-amino,

$R^3$ represents hydrogen, hydroxyl, halogen, $C_1$-$C_4$-alkyl, $C_1$-$C_4$-halogenoalkyl, $C_1$-$C_4$-alkoxy or $C_1$-$C_4$-halogenoalkoxy,

$R^4$ represents hydrogen or $C_1$-$C_4$-alkyl.

**7.** Process for preparing substituted pyridines of the formula (I) according to Claim 1, in which

A represents nitrogen or a CH grouping,

Q represents oxygen,

$R^1$ represents hydrogen, chlorine, methyl, ethyl, trifluoromethyl, methoxymethyl, methoxy, ethoxy, difluoromethoxy, methylthio, methylamino, ethylamino or dimethylamino,

$R^2$ represents hydrogen, chlorine, methyl, ethyl, trifluoromethyl, methoxy, ethoxy, difluoromethoxy, methylthio, methylamino, ethylamino or dimethylamino,

$R^3$ represents hydrogen, hydroxyl, fluorine, chlorine, bromine, methyl, ethyl, trifluoromethyl, methoxy, difluoromethoxy, trifluoromethoxy,

$R^4$ represents hydrogen or methyl, and

Z represents one of the following groupings:

N-$R^5$ or

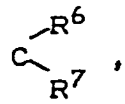

where

$R^5$ represents hydrogen, hydroxyl or amino, represents methyl, ethyl, n-propyl, isopropyl, n-butyl, isobutyl, sec-butyl, tert-butyl, allyl, propargyl, methoxy, ethoxy, n-propoxy, isopropoxy, n-butoxy, isobutoxy, sec-butoxy, allyloxy, methoxycarbonylmethoxy, ethoxycarbonylmethoxy, methoxycarbonylethoxy, ethoxycarbonylethoxy, methylamino, ethylamino, n-propylamino, isopropylamino, n-butylamino, isobutylamino, sec-butylamino, tert-butylamino, dimethylamino, acetylamino, propionylamino, methoxycarbonylamino, ethoxycarbonylamino, methylsulphonylamino or ethylsulphonylamino, represents phenyl, benzyl, phenoxy, benzyloxy, phenylamino, benzylamino, N-methyl-N-phenylamino, pyridylamino, pyrimidylamino, pyridylcarbonylamino, phenylcarbonylamino, furylcarbonylamino, thienylcarbonylamino or phenylsulphonylamino, each of which is optionally substituted by nitro, cyano, fluorine, chlorine, bromine, methyl, trifluoromethyl, methoxy, trifluoromethoxy, methylthio or trifluoromethylthio,

$R^6$ represents hydrogen, fluorine, chlorine, cyano, carboxyl, $C_1$-$C_4$-alkoxy-carbonyl, $C_1$-$C_4$-alkylcarbonylamino, dimethoxyphosphoryl or diethoxyphosphoryl, and

$R^7$ represents formyl, cyano, carboxyl, hydroxymethyl carbamoyl, represents $C_1$-$C_4$-alkoxy-carbonyl, $C_5$-$C_6$-cycloalkyloxy-carbonyl, $C_1$-$C_4$-alkylthio-carbonyl, $C_1$-$C_4$-alkylamino-carbonyl or $C_5$-$C_6$-cycloalkylamino-carbonyl, each of which is optionally substituted by fluorine, chlorine, carboxyl or $C_1$-$C_4$-alkoxy-carbonyl, represents dimethylaminocarbonyl, represents $C_1$-$C_4$-alkylaminocarbonyl-$C_1$-$C_4$-alkoxy-carbonyl, represents dimethylaminocarbonyl-$C_1$-$C_4$-alkoxycarbonyl, represents N-methyl-N-phenylaminocarbonyl-$C_1$-$C_4$-alkoxy-carbonyl, represents pyrrolidinylcarbonyl, piperidinyl-carbonyl, morpholinylcarbonyl or piperazinyl-carbonyl, each of which is optionally substituted by methyl and/or ethyl, represents phenoxycarbonyl, benzyloxycarbonyl, phenylthiocarbonyl, benzylthiocarbonyl, phenylaminocarbonyl, benzylaminocarbonyl, N-methyl-N-phenylaminocarbonyl or phenylhydrazinocarbonyl, each of which is optionally substituted by nitro, cyano, fluorine, chlorine, bromine, methyl, trifluoromethyl, methoxy, trifluoromethoxy, methylthio or trifluoromethylthio, represents phthalimidoxycarbonyl, or, together with $R^6$, represents the grouping $-CO-O-CH_2CH_2-$.

**8.** Process for preparing substituted pyridines of the formula (I) according to Claim 1, in which

A represents a CH grouping,
Q represents oxygen,
$R^1$ represents methoxy,
$R^2$ represents methoxy,
$R^3$ represents hydrogen or fluorine,
$R^4$ represents hydrogen, and
Z has the meaning given in Claim 7.

**Revendications**

**Revendications pour les Etats contractants suivants : BE, CH, DE, FR, GB, IT, LI, NL**

**1.** Pyridines substituées de formule générale I :

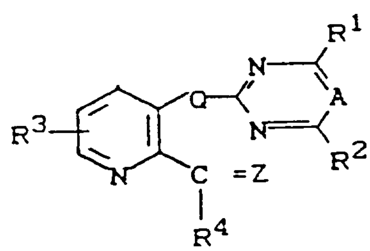

( I )

dans laquelle
A représente l'azote ou un groupement C-X dans lequel X représente l'hydrogène ou un halogène,
Q représente l'oxygène ou le soufre,
$R^1$ et $R^2$, ayant des significations identiques ou différentes, représentent chacun l'hydrogène, un halogène, un groupe alkyle en $C_1$-$C_4$, halogénoalkyle en $C_1$-$C_4$, (alcoxy en $C_1$-$C_2$)alkyle en $C_1$-$C_2$, alcoxy en $C_1$-$C_4$, halogénoalcoxy en $C_1$-$C_4$, alkylthio en $C_1$-$C_4$, alkylamino en $C_1$-$C_4$ ou di(alkyle en $C_1$-$C_2$)amino,
$R^3$ représente l'hydrogène, un groupe hydroxy, un halogène, un groupe alkyle en $C_1$-$C_4$, halogénoalkyle en $C_1$-$C_4$, alcoxy en $C_1$-$C_4$, halogénoalcoxy en $C_1$-$C_4$,
$R^4$ représente l'hydrogène ou un groupe alkyle en $C_1$-$C_4$, et Z représente l'un des groupements suivants :
N-$R^5$ ou

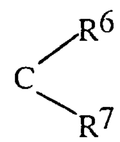

dans lesquels
$R^5$ représente l'hydrogène, un groupe hydroxy, amino ou un groupe alkyle en $C_1$-$C_6$, alcényle en $C_3$-$C_6$, alcynyle en $C_3$-$C_6$, alcoxy en $C_1$-$C_6$, alcényloxy en $C_3$-$C_6$, (alcoxy en $C_1$-$C_4$)carbonylalcoxy en $C_1$-$C_2$, alkylamino en $C_1$-$C_6$, di(alkyle en $C_1$-$C_2$)amino, (alkyle en $C_1$-$C_6$)carbonylamino, (alcoxy en $C_1$-$C_6$)-carbonylamino, alkylsulfonylamino en $C_1$-$C_6$, chacun d'eux éventuellement substitué par des halogènes, ou bien un groupe phényle, phénylalkyle en $C_1$-$C_4$, phénoxy, phénylalcoxy en $C_1$-$C_4$, phénylamino, phénylalkylamino en $C_1$-$C_4$, N-(alkyle en $C_1$-$C_4$)-N-phénylamino, pyridylamino, pyrimidylamino, pyridylcarbonylamino, phénylcarbonylamino, furylcarbonylamino, thiénylcarbonylamino ou phénylsulfonylamino, chacun d'eux éventuellement substitué par des groupes nitro, amino, cyano, carboxy, des halogènes, des groupes alkyle en $C_1$-$C_4$, halogénoalkyle en $C_1$-$C_2$, alcoxy en $C_1$-$C_4$, halogénoalcoxy en $C_1$-$C_2$, alkylthio en $C_1$-$C_4$, halogénoalkylthio en $C_1$-$C_2$, (alcoxy en $C_1$-$C_4$)carbonyle et/ou di(alkyle en $C_1$-$C_2$)amino,
$R^6$ représente l'hydrogène, un halogène, un groupe cyano, carboxy, (alcoxy en $C_1$-$C_6$)carbonyle, (alkyle en $C_1$-$c_6$)carbonylamino ou di(alcoxy en $C_1$-$C_4$)-phosphoryle, et
$R^7$ représente un groupe formyle, cyano, carboxy, hydroxyméthyle, carbamoyle, ou bien un groupe (alcoxy en $C_1$-$C_6$)carbonyle, (cycloalkyloxy en $C_5$-$C_6$)-carbonyle, (alkylthio en $C_1$-$C_6$)carbonyle, (alkylamino en $C_1$-$C_6$)carbonyle ou (cycloalkylamino en $C_5$-$C_6$)carbonyle, chacun d'eux éventuellement substitué par des halogènes, des groupes carboxy ou (alcoxy en $C_1$-$C_4$)carbonyle, un groupe di(alkyle en $C_1$-$C_2$)aminocarbonyle, un groupe (alkylamino en $C_1$-$C_4$)carbonyl-(alcoxy en $C_1$-$C_4$)carbonyle, un groupe di(alkyle en $C_1$-$C_2$)aminocarbonyl(alcoxy en $C_1$-$C_4$)carbonyle, un groupe phénylaminocarbonyl-(alcoxy en $C_1$-$C_4$)carbonyle, un groupe N-méthyl-N-phénylaminocarbonyl(alcoxy en $C_1$-$C_4$)carbonyle, ou bien un groupe pyrrolidinylcarbonyle, pipéridinylcarbonyle, morpholinylcarbonyle ou pipérazinylcarbonyle, chacun d'eux éventuellement substitué par des groupes méthyle et/ou éthyle, un groupe phénoxycarbonyle, phényl(alcoxy en $C_1$-$C_4$)carbonyle, furylméthoxycarbonyle, thiénylméthoxycarbonyle, phénylthiocarbonyle, phényl(alkylthio en $C_1$-$C_4$)carbonyle, phénylaminocarbonyle, phényl(alkylamino en $C_1$-$C_4$)carbonyle, N-(alkyle en $C_1$-$C_4$)-N-phénylaminocarbonyle ou phénylhydrazinocarbonyle ou (alkyle en $C_1$-$C_4$)-hydrazinocarbonyle, chacun d'eux éventuellement substitué par des groupes nitro, amino, cyano, carboxy, des halogènes, des groupes alkyle en $C_1$-$C_4$, halogénoalkyle en $C_1$-$C_2$, alcoxy en $C_1$-$C_4$, halogénoalcoxy en $C_1$-$C_2$, alkylthio en $C_1$-$C_4$, halogénoalkylthio en $C_1$-$C_2$, (alcoxy en $C_1$-$C_4$)-carbonyle et/ou di(alkyle en $C_1$-$C_2$)amino, un groupe phtalimidoxycarbonyle, ou bien
$R^7$ et $R^6$ forment ensemble le groupement $-CO-O-(CH_2)_n-$ dans lequel n est un nombre allant de 1 à 4.

**2.** Pyridines substituées de formule I selon revendication 1, dans laquelle A représente l'azote ou le groupement CH,
Q représente l'oxygène,
$R^1$ représente l'hydrogène, le chlore, un groupe méthyle, éthyle, trifluorométhyle, méthoxyméthyle, méthoxy, éthoxy, difluorométhoxy, méthylthio, méthylamino, éthylamino ou diméthylamino,
$R^2$ représente l'hydrogène, le chlore, un groupe méthyle, éthyle, trifluorométhyle, méthoxy, éthoxy, difluorométhoxy, méthylthio, méthylamino, éthylamino ou diméthylamino,

$R^3$ représente l'hydrogène, un groupe hydroxy, le fluor, le chlore, le brome, un groupe méthyle, éthyle, trifluorométhyle, méthoxy, difluorométhoxy, trifluorométhoxy,
$R^4$ représente l'hydrogène ou un groupe méthyle, et
Z représente l'un des groupements suivants :

N-$R^5$ ou

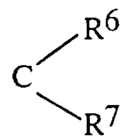

dans lesquels
$R^5$ représente l'hydrogène, un groupe hydroxy, amino, un groupe méthyle, éthyle, n-propyle, isopropyle, n-butyle, isobutyle, sec-butyle, tert-butyle, allyle, propargyle, méthoxy, éthoxy, n-propoxy, isopropoxy, n-butoxy, isobutoxy, sec-butoxy, allyloxy, méthoxycarbonylméthoxy, éthoxycarbonylméthoxy, méthoxy-carbonyléthoxy, éthoxycarbonyléthoxy, méthylamino, éthylamino, n-propylamino, isopropylamino, n-butylamino, isobutylamino, sec-butylamino, tert-butylamino, diméthylamino, acétylamino, propionylamino, méthoxy-carbonylamino, éthoxycarbonylamino, méthylsulfonylamino, éthylsulfonylamino, un groupe phényle, benzyle, phénoxy, benzyloxy, phénylamino, benzylamino, N-méthyl-N-phénylamino, pyridylamino, pyrimidylamino, pyridylcarbonylamino, phénylcarbonylamino, furylcarbonylamino, thiénylcarbonylamino ou phénylsulfonylamino, chacun d'eux éventuellement substitué par des groupes nitro, cyano, le fluor, le chlore, le brome, des groupes méthyle, trifluorométhyle, méthoxy, trifluorométhoxy, méthylthio ou trifluorométhylthio,
$R^6$ représente l'hydrogène, le fluor, le chlore, un groupe cyano, carboxy, (alcoxy en $C_1$-$C_4$)carbonyle, (alkyle en $C_1$-$C_4$)carbonylamino, diméthoxyphosphoryle ou diéthoxyphosphoryle, et
$R^7$ représente un groupe formyle, cyano, carboxy, hydroxyméthyle, carbamoyle, un groupe (alcoxy en $C_1$-$C_4$)carbonyle, (cycloalkyloxy en $C_5$-$C_6$)carbonyle, (alkylthio en $C_1$-$C_4$)carbonyle, (alkylamino en $C_1$-$C_4$)carbonyle ou (cycloalkylamino en $C_5$-$C_6$)carbonyle, chacun d'eux éventuellement substitué par le fluor, le chlore, des groupes carboxy ou (alcoxy en $C_1$-$C_4$)carbonyle, un groupe diméthylaminocarbonyle, (alkylamino en $C_1$-$C_4$)carbonyl(alcoxy en $C_1$-$C_4$)carbonyle, un groupe diméthylaminocarbonyle (alcoxy en $C_1$-$C_4$)carbonyle, un groupe N-méthyl-N-phénylaminocarbonyl(alcoxy en $C_1$-$C_4$)carbonyle ou bien un groupe pyrrolidinylcarbonyle, pipéridinylcarbonyle, morpholinylcarbonyle ou pipérazinylcarbonyle, chacun d'eux éventuellement substitué par des groupes méthyle et/ou éthyle, ou un groupe phénoxycarbonyle, benzyloxycarbonyle, phénylthiocarbonyle, benzylthiocarbonyle, phénylaminocarbonyle, benzylaminocarbonyle, N-méthyl-N-phénylaminocarbonyle, phénylhydrazinocarbonyle, chacun d'eux éventuellement substitué par des groupes nitro, cyano, le fluor, le chlore, le brome, des groupes méthyle, trifluorométhyle, méthoxy, trifluorométhoxy, méthylthio ou trifluorométhylthio, ou un groupe phtalimidoxycarbonyle ou bien
$R^7$ et $R^6$ forment ensemble le groupement $-CO-O-CH_2CH_2-$.

**3.** Pyridines substituées de formule I de la revendication 1, dans laquelle A représente un groupement CH,
Q représente l'oxygène,
$R^1$ représente un groupe méthoxy,
$R^2$ représente un groupe méthoxy,
$R^3$ représente l'hydrogène ou le fluor,
$R^4$ représente l'hydrogène, et
Z a les significations indiquées dans la revendication 2.

**4.** Procédé de préparation des pyridines substituées de formule I :

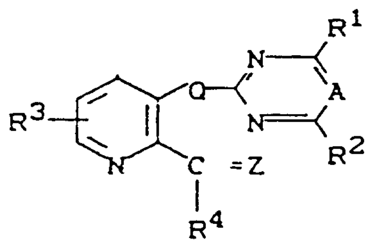

( I )

dans laquelle
A représente l'azote ou un groupement C-X dans lequel X représente l'hydrogène ou un halogène,
Q représente l'oxygène ou le soufre,
$R^1$ et $R^2$, ayant des significations identiques ou différentes, représentent chacun l'hydrogène, un halogène, un groupe alkyle en $C_1$-$C_4$, halogénoalkyle en $C_1$-$C_4$, (alcoxy en $C_1$-$C_2$)alkyle en $C_1$-$C_2$, alcoxy en $C_1$-$C_4$, halogénoalcoxy en $C_1$-$C_4$, alkylthio en $C_1$-$C_4$, alkylamino en $C_1$-$C_4$ ou di(alkyle en $C_1$-$C_2$)amino,
$R^3$ représente l'hydrogène, un groupe hydroxy, un halogène, un groupe alkyle en $C_1$-$C_4$, halogénoalkyle en $C_1$-$C_4$, alcoxy en $C_1$-$C_4$, halogénoalcoxy en $C_1$-$C_4$,
$R^4$ représente l'hydrogène ou un groupe alkyle en $C_1$-$C_4$, et
Z représente l'un des groupements suivants :
N-$R^5$ ou

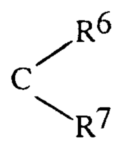

dans lesquels
$R^5$ représente l'hydrogène, un groupe hydroxy, amino ou un groupe alkyle en $C_1$-$C_6$, alcényle en $C_3$-$C_6$, alcynyle en $C_3$-$C_6$, alcoxy en $C_1$-$C_6$, alcényloxy en $C_3$-$C_6$, (alcoxy en $C_1$-$C_4$)carbonylalcoxy en $C_1$-$C_2$, alkylamino en $C_1$-$C_6$, di(alkyle en $C_1$-$C_2$)amino, (alkyle en $C_1$-$C_6$)carbonylamino, (alcoxy en $C_1$-$C_6$)-carbonylamino, alkylsulfonylamino en $C_1$-$C_6$, chacun d'eux éventuellement substitué par des halogènes, ou bien un groupe phényle, phénylalkyle en $C_1$-$C_4$, phénoxy, phénylalcoxy en $C_1$-$C_4$, phénylamino, phénylalkylamino en $C_1$-$C_4$, N-(alkyle en $C_1$-$C_4$)-N-phénylamino, pyridylamino, pyrimidylamino, pyridylcarbonylamino, phénylcarbonylamino, furylcarbonylamino, thiénylcarbonylamino ou phénylsulfonylamino, chacun d'eux éventuellement substitué par des groupes nitro, amino, cyano, carboxy, des halogènes, des groupes alkyle en $C_1$-$C_4$, halogénoalkyle en $C_1$-$C_2$, alcoxy en $C_1$-$C_4$, halogénoalcoxy en $C_1$-$C_2$, alkylthio en $C_1$-$C_4$, halogénoalkylthio en $C_1$-$C_2$, (alcoxy en $C_1$-$C_4$)carbonyle et/ou di(alkyle en $C_1$-$C_2$)amino,
$R^6$ représente l'hydrogène, un halogène, un groupe cyano, carboxy, (alcoxy en $C_1$-$C_6$)carbonyle, (alkyle en $C_1$-$c_6$)carbonylamino ou di(alcoxy en $C_1$-$C_4$)-phosphoryle, et
$R^7$ représente un groupe formyle, cyano, carboxy, hydroxyméthyle, carbamoyle, ou bien un groupe (alcoxy en $C_1$-$C_6$)carbonyle, (cycloalkyloxy en $C_5$-$C_6$)-carbonyle, (alkylthio en $C_1$-$C_6$)carbonyle, (alkylamino en $C_1$-$C_6$)carbonyle ou (cycloalkylamino en $C_5$-$C_6$)carbonyle, chacun d'eux éventuellement substitué par des halogènes, des groupes carboxy ou (alcoxy en $C_1$-$C_4$)carbonyle, un groupe di(alkyle en $C_1$-$C_2$)aminocarbonyle, un groupe (alkylamino en $C_1$-$C_4$)carbonyl-(alcoxy en $C_1$-$C_4$)carbonyle, un groupe di(alkyle en $C_1$-$C_2$)aminocarbonyl(alcoxy en $C_1$-$C_4$)carbonyle, un groupe phénylaminocarbonyl-(alcoxy en $C_1$-$C_4$)carbonyle, un groupe N-méthyl-N-phénylaminocarbonyl(alcoxy en $C_1$-$C_4$)carbonyle, ou bien un groupe pyrrolidinylcarbonyle, pipéridinylcarbonyle, morpholinylcarbonyle ou pipérazinylcarbonyle, chacun d'eux éventuellement substitué par des groupes méthyle et/ou éthyle, un groupe phénoxycarbonyle, phényl(alcoxy en $C_1$-$C_4$)carbonyle, furylméthoxycarbonyle, thiénylméthoxycarbonyle, phénylthiocarbonyle, phényl(alkylthio en $C_1$-$C_4$)carbonyle, phénylaminocarbonyle, phényl(alkylamino en $C_1$-$C_4$)carbonyle, N-(alkyle en $C_1$-$C_4$)-N-phénylaminocarbonyle ou phénylhydrazinocarbonyle ou (alkyle en $C_1$-$C_4$)-hydrazinocarbonyle, chacun d'eux éventuellement substitué par des groupes nitro,

amino, cyano, carboxy, des halogènes, des groupes alkyle en $C_1$-$C_4$, halogénoalkyle en $C_1$-$C_2$, alcoxy en $C_1$-$C_4$, halogénoalcoxy en $C_1$-$C_2$, alkylthio en $C_1$-$C_4$, halogénoalkylthio en $C_1$-$C_2$, (alcoxy en $C_1$-$C_4$)-carbonyle et/ou di(alkyle en $C_1$-$C_2$)amino, un groupe phtalimidoxycarbonyle, ou bien

$R^7$ et $R^6$ forment ensemble le groupement $-CO-O-(CH_2)_n-$ dans lequel n est un nombre allant de 1 à 4,

caractérisé en ce que l'on fait réagir des pyridines substituées de formule générale Ia :

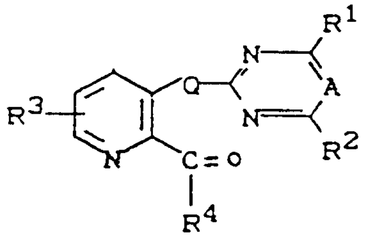

(Ia)

dans laquelle

A, Q, $R^1$, $R^2$, $R^3$ et $R^4$ ont les significations indiquées ci-dessus, avec des dérivés aminés ou méthylénés de formule générale III :

$H_2Z$ (III)

dans laquelle

Z a les significations indiquées ci-dessus,

éventuellement en présence d'un produit auxiliaire de réaction et éventuellement en présence d'un diluant.

5. Produits herbicides, caractérisés en ce qu'ils contiennent au moins une pyridine substituée de formule I selon les revendications 1 à 3.

6. Procédé pour combattre les croissances de végétaux indésirables, caractérisé en ce que l'on fait agir des pyridines substituées de formule I selon les revendications 1 à 3 sur les végétaux indésirables et/ou leur habitat.

7. Utilisation des pyridines substituées de formule I selon les revendications 1 à 3, pour la lutte contre les végétaux indésirables.

8. Procédé de préparation de produits herbicides, caractérisé en ce que l'on mélange des pyridines substituées de formule I selon les revendications 1 à 3, avec des diluants et/ou des agents tensioactifs.

9. Hydroxyalkylpyridines de formule générale II :

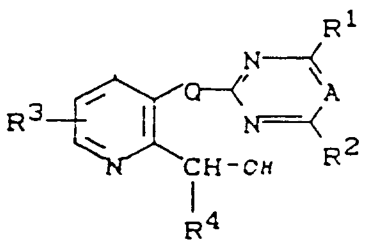

(II)

dans laquelle

A représente l'azote ou un groupement C-X dans lequel X représente l'hydrogène ou un halogène,

Q représente l'oxygène ou le soufre,

$R^1$ et $R^2$, ayant des significations identiques ou différentes, représentent chacun l'hydrogène, un halogène, un groupe alkyle en $C_1$-$C_4$, halogénoalkyle en $C_1$-$C_4$, (alcoxy en $C_1$-$C_2$)alkyle en $C_1$-$C_2$, alcoxy en $C_1$-$C_4$, halogénoalcoxy en $C_1$-$C_4$, alkylthio en $C_1$-$C_4$, alkylamino en $C_1$-$C_4$ ou di(alkyle en $C_1$-

$C_2$)amino,
$R^3$ représente l'hydrogène, un groupe hydroxy, des halogènes, un groupe alkyle en $C_1$-$C_4$, halogénoalkyle en $C_1$-$C_4$, alcoxy en $C_1$-$C_4$, halogénoalcoxy en $C_1$-$C_4$,
$R^4$ représente l'hydrogène ou un groupe alkyle en $C_1$-$C_4$.

**Revendications pour l'Etat contractant suivant : ES**

**1.** Procédé de préparation de pyridines substituées de formule générale I :

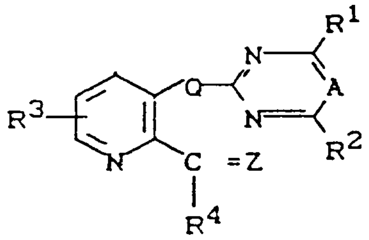

(I)

dans laquelle
A représente l'azote ou un groupement C-X dans lequel X représente l'hydrogène ou un halogène,
Q représente l'oxygène ou le soufre,
$R^1$ et $R^2$, ayant des significations identiques ou différentes, représentent chacun l'hydrogène, un halogène, un groupe alkyle en $C_1$-$C_4$, halogénoalkyle en $C_1$-$C_4$, (alcoxy en $C_1$-$C_2$)alkyle en $C_1$-$C_2$, alcoxy en $C_1$-$C_4$, halogénoalcoxy en $C_1$-$C_4$, alkylthio en $C_1$-$C_4$, alkylamino en $C_1$-$C_4$ ou di(alkyle en $C_1$-$C_2$)amino,
$R^3$ représente l'hydrogène, un groupe hydroxy, un halogène, un groupe alkyle en $C_1$-$C_4$, halogénoalkyle en $C_1$-$C_4$, alcoxy en $C_1$-$C_4$, halogénoalcoxy en $C_1$-$C_4$,
$R^4$ représente l'hydrogène ou un groupe alkyle en $C_1$-$C_4$, et
Z représente l'un des groupements suivants :
N-$R^5$ ou

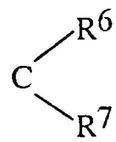

dans lesquels
$R^5$ représente l'hydrogène, un groupe hydroxy, amino ou un groupe alkyle en $C_1$-$C_6$, alcényle en $C_3$-$C_6$, alcynyle en $C_3$-$C_6$, alcoxy en $C_1$-$C_6$, alcényloxy en $C_3$-$C_6$, (alcoxy en $C_1$-$C_4$)carbonylalcoxy en $C_1$-$C_2$, alkylamino en $C_1$-$C_6$, di(alkyle en $C_1$-$C_2$)amino, (alkyle en $C_1$-$C_6$)carbonylamino, (alcoxy en $C_1$-$C_6$)-carbonylamino, alkylsulfonylamino en $C_1$-$C_6$, chacun d'eux éventuellement substitué par des halogènes, ou bien un groupe phényle, phénylalkyle en $C_1$-$C_4$, phénoxy, phénylalcoxy en $C_1$-$C_4$, phénylamino, phénylalkylamino en $C_1$-$C_4$, N-(alkyle en $C_1$-$C_4$)-N-phénylamino, pyridylamino, pyrimidylamino, pyridylcarbonylamino, phénylcarbonylamino, furylcarbonylamino, thiénylcarbonylamino ou phénylsulfonylamino, chacun d'eux éventuellement substitué par des groupes nitro, amino, cyano, carboxy, des halogènes, des groupes alkyle en $C_1$-$C_4$, halogénoalkyle en $C_1$-$C_2$, alcoxy en $C_1$-$C_4$, halogénoalcoxy en $C_1$-$C_2$, alkylthio en $C_1$-$C_4$, halogénoalkylthio en $C_1$-$C_2$, (alcoxy en $C_1$-$C_4$)carbonyle et/ou di(alkyle en $C_1$-$C_2$)amino,
$R^6$ représente l'hydrogène, un halogène, un groupe cyano, carboxy, (alcoxy en $C_1$-$C_6$)carbonyle, (alkyle en $C_1$-$C_6$)carbonylamino ou di(alcoxy en $C_1$-$C_4$)-phosphoryle, et
$R^7$ représente un groupe formyle, cyano, carboxy, hydroxyméthyle, carbamoyle, ou bien un groupe (alcoxy en $C_1$-$C_6$)carbonyle, (cycloalkyloxy en $C_5$-$C_6$)carbonyle, (alkylthio en $C_1$-$C_6$)carbonyle, (alkylamino en $C_1$-$C_6$)carbonyle ou (cycloalkylamino en $C_5$-$C_6$)carbonyle, chacun d'eux éventuellement substitué par des halogènes, des groupes carboxy ou (alcoxy en $C_1$-$C_4$)carbonyle, un groupe di(alkyle en $C_1$-$C_2$)aminocarbonyle, un groupe (alkylamino en $C_1$-$C_4$)carbonyl(alcoxy en $C_1$-$C_4$)carbonyle, un groupe di(alkyle en $C_1$-$C_2$)aminocarbonyl(alcoxy en $C_1$-$C_4$)carbonyle, un groupe phénylaminocarbonyl-

(alcoxy en $C_1$-$C_4$)carbonyle, un groupe N-méthyl-N-phénylaminocarbonyl(alcoxy en $C_1$-$C_4$)carbonyle, ou bien un groupe pyrrolidinylcarbonyle, pipéridinylcarbonyle, morpholinylcarbonyle ou pipérazinylcarbonyle, chacun d'eux éventuellement substitué par des groupes méthyle et/ou éthyle, un groupe phénoxycarbonyle, phényl(alcoxy en $C_1$-$C_4$)carbonyle, furylméthoxycarbonyle, thiénylméthoxycarbonyle, phénylthiocarbonyle, phényl(alkylthio en $C_1$-$C_4$)carbonyle, phénylaminocarbonyle, phényl(alkylamino en $C_1$-$C_4$)carbonyle, N-(alkyle en $C_1$-$C_4$)-N-phénylaminocarbonyle ou phénylhydrazinocarbonyle ou (alkyle en $C_1$-$C_4$)-hydrazinocarbonyle, chacun d'eux éventuellement substitué par des groupes nitro, amino, cyano, carboxy, des halogènes, des groupes alkyle en $C_1$-$C_4$, halogénoalkyle en $C_1$-$C_2$, alcoxy en $C_1$-$C_4$, halogénoalcoxy en $C_1$-$C_2$, alkylthio en $C_1$-$C_4$, halogénoalkylthio en $C_1$-$C_2$, (alcoxy en $C_1$-$C_4$)-carbonyle et/ou di(alkyle en $C_1$-$C_2$)amino, un groupe phtalimidoxycarbonyle, ou bien
$R^7$ et $R^6$ forment ensemble le groupement $-CO-O-(CH_2)_n-$ dans lequel n est un nombre allant de 1 à 4,
caractérisé en ce que l'on fait réagir des pyridines substituées de formule générale Ia :

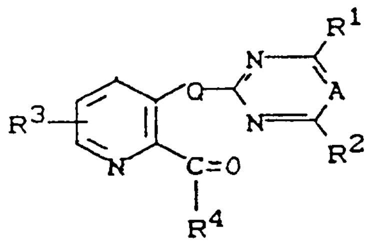
(Ia)

dans laquelle
A, Q, $R^1$, $R^2$, $R^3$ et $R^4$ ont les significations indiquées ci-dessus, avec des dérivés aminés ou méthylénés de formule générale III :

$H_2Z$ (III)

dans laquelle
Z a les significations indiquées ci-dessus,
éventuellement en présence d'un produit auxiliaire de réaction et éventuellement en présence d'un diluant.

2. Produits herbicides, caractérisés en ce qu'ils contiennent au moins une pyridine substituée de formule I de la revendication 1.

3. Procédé pour combattre les croissances de végétaux indésirables, caractérisé en ce que l'on fait agir des pyridines substituées de formule I de la revendication 1 sur les végétaux indésirables et/ou leur habitat.

4. Utilisation des pyridines substituées de formule I de la revendication 1 pour la lutte contre les croissances de végétaux indésirables.

5. Procédé de préparation de produits herbicides, caractérisé en ce que l'on mélange des pyridines substituées de formule I de la revendication 1 avec des diluants et/ou des agents tensioactifs.

6. Procédé de préparation des hydroxyalkylpyridines de formule générale II :

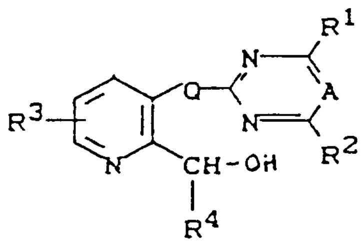
(II)

dans laquelle
A représente l'azote ou un groupement C-X dans lequel X représente l'hydrogène ou un halogène,
Q représente l'oxygène ou le soufre,
$R^1$ et $R^2$, ayant des significations identiques ou différentes, représentent chacun l'hydrogène, un halogène, un groupe alkyle en $C_1$-$C_4$, halogénoalkyle en $C_1$-$C_4$, (alcoxy en $C_1$-$C_2$)alkyle en $C_1$-$C_2$, alcoxy en $C_1$-$C_4$, halogénoalcoxy en $C_1$-$C_4$, alkylthio en $C_1$-$C_4$, alkylamino en $C_1$-$C_4$ ou di(alkyle en $C_1$-$C_2$)amino,
$R^3$ représente l'hydrogène, un groupe hydroxy, des halogènes, un groupe alkyle en $C_1$-$C_4$, halogénoalkyle en $C_1$-$C_4$, alcoxy en $C_1$-$C_4$, halogénoalcoxy en $C_1$-$C_4$, $R^4$ représente l'hydrogène ou un groupe alkyle en $C_1$-$C_4$.

**7.** Procédé de préparation des pyridines substituées de formule I de la revendication 1, dans laquelle
A représente l'azote ou le groupement CH,
Q représente l'oxygène,
$R^1$ représente l'hydrogène, le chlore, un groupe méthyle, éthyle, trifluorométhyle, méthoxyméthyle, méthoxy, éthoxy, difluorométhoxy, méthylthio, méthylamino, éthylamino ou diméthylamino,
$R^2$ représente l'hydrogène, le chlore, un groupe méthyle, éthyle, trifluorométhyle, méthoxy, éthoxy, difluorométhoxy, méthylthio, méthylamino, éthylamino ou diméthylamino,
$R^3$ représente l'hydrogène, un groupe hydroxy, le fluor, le chlore, le brome, un groupe méthyle, éthyle, trifluorométhyle, méthoxy, difluorométhoxy, trifluorométhoxy,
$R^4$ représente l'hydrogène ou un groupe méthyle, et
Z représente l'un des groupements suivants :
N-$R^5$ ou

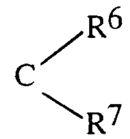

dans lesquels
$R^5$ représente l'hydrogène, un groupe hydroxy, amino, un groupe méthyle, éthyle, n-propyle, isopropyle, n-butyle, isobutyle, sec-butyle, tert-butyle, allyle, propargyle, méthoxy, éthoxy, n-propoxy, isopropoxy, n-butoxy, isobutoxy, sec-butoxy, allyloxy, méthoxycarbonylméthoxy, éthoxycarbonylméthoxy, méthoxycarbonyléthoxy, éthoxycarbonyléthoxy, méthylamino, éthylamino, n-propylamino, isopropylamino, n-butylamino, isobutylamino, sec-butylamino, tert-butylamino, diméthylanlino, acétylamino, propionylamino, méthoxycarbonylamino, éthoxycarbonylamino, méthylsulfonylamino, éthylsulfonylamino, un groupe phényle, benzyle, phénoxy, benzyloxy, phénylamino, benzylamino, N-méthyl-N-phénylamino, pyridylamino, pyrimidylamino, pyridylcarbonylamino, phénylcarbonylamino, furylcarbonylamino, thiénylcarbonylamino ou phénylsulfonylamino, chacun d'eux éventuellement substitué par des groupes nitro, cyano, le fluor, le chlore, le brome, des groupes méthyle, trifluorométhyle, méthoxy, trifluorométhoxy, méthylthio ou trifluorométhylthio,
$R^6$ représente l'hydrogène, le fluor, le chlore, un groupe cyano, carboxy, (alcoxy en $C_1$-$C_4$)carbonyle, (alkyle en $C_1$-$C_4$)carbonylamino, diméthoxyphosphoryle ou diéthoxyphosphoryle, et
$R^7$ représente un groupe formyle, cyano, carboxy, hydroxyméthyle, carbamoyle, un groupe (alcoxy en $C_1$-$C_4$)carbonyle, (cycloalkyloxy en $C_5$-$C_6$)carbonyle, (alkylthio en $C_1$-$C_4$)carbonyle, (alkylamino en $C_1$-$C_4$)carbonyle ou (cycloalkylamino en $C_5$-$C_6$)carbonyle, chacun d'eux éventuellement substitué par le fluor, le chlore, des groupes carboxy ou (alcoxy en $C_1$-$C_4$)carbonyle, un groupe diméthylaminocarbonyle, (alkylamino en $C_1$-$C_4$)carbonyl(alcoxy en $C_1$-$C_4$)carbonyle, un groupe diméthylaminocarbonyle (alcoxy en $C_1$-$C_4$)carbonyle, un groupe N-méthyl-N-phénylaminocarbonyl(alcoxy en $C_1$-$C_4$)carbonyle ou bien un groupe pyrrolidinylcarbonyle, pipéridinylcarbonyle, morpholinylcarbonyle ou pipérazinylcarbonyle, chacun d'eux éventuellement substitué par des groupes méthyle et/ou éthyle, ou un groupe phénoxycarbonyle, benzyloxycarbonyle, phénylthiocarbonyle, benzylthiocarbonyle, phénylaminocarbonyle, benzylaminocarbonyle, N-méthyl-N-phénylaminocarbonyle, phénylhydrazinocarbonyle, chacun d'eux éventuellement substitué par des groupes nitro, cyano, le fluor, le chlore, le brome, des groupes méthyle, trifluorométhyle, méthoxy, trifluorométhoxy, méthylthio ou trifluorométhylthio, ou un groupe phtalimidoxycarbonyle ou bien
$R^7$ et $R^6$ forment ensemble le groupement $-CO-O-CH_2CH_2-$.

**8.** Procédé de préparation des pyridines substituées de formule I de la revendication 1, dans laquelle
A représente un groupement CH,
Q représente l'oxygène,
$R^1$ représente un groupe méthoxy,
$R^2$ représente un groupe méthoxy,
$R^3$ représente l'hydrogène ou le fluor,
$R^4$ représente l'hydrogène, et
Z a les significations indiquées dans la revendication 7.